# EUROPÄISCHE PATENTSCHRIFT

(11) **EP 3 322 817 B1**
(45) Veröffentlichungstag und Bekanntmachung des Hinweises auf die Patenterteilung: **02.12.2020**
(21) Anmeldenummer: 16739091.3
(22) Anmeldetag: 12.07.2016
(51) Int. Cl.: C12Q 1/6883, C12Q 1/6893

(54) **OLIGONUCLEOTIDE UND DEREN VERWENDUNG**
OLIGONUCLEOTIDES AND USE THEREOF
OLIGONUCLÉOTIDES ET LEUR UTILISATION

(30) Priorität: 13.07.2015 DE 102015111267; 07.08.2015 DE 102015113038
(43) Veröffentlichungstag der Anmeldung: 23.05.2018
(73) Patentinhaber: Kann, Simone, 53721 Siegburg (DE)
(72) Erfinder: HANSEN, Jessica, 22159 Hamburg (DE); SIEVERTSEN, Juergen, 20359 Hamburg (DE); KANN, Simone, 53721 Siegburg (DE)
(74) Vertreter: Wichmann, Hendrik
(86) Internationale Anmeldenummer: PCT/EP2016/066561
(87) Internationale Veröffentlichungsnummer: WO 2017/009347

(56) Entgegenhaltungen:
- WO-A2-01/16312
- WO-A2-2011/087782
- WO-A2-2015/006755
- CA-A1- 2 736 087
- US-A1- 2013 267 429
- ELIZABETH A. GARCÍA ET AL: "TcTASV: A Novel Protein Family in Trypanosoma cruzi Identified from a Subtractive Trypomastigote cDNA Library", PLOS NEGLECTED TROPICAL DISEASES, Bd. 4, Nr. 10, 5. Oktober 2010 (2010-10-05), Seite e841, XP055309452, DOI: 10.1371/journal.pntd.0000841 & DATABASE EMBL [Online] 16. Mai 2010 (2010-05-16), "TcT-E01h14.b1d TcT-E Trypanosoma cruzi cDNA clone tcte-01h14, mRNA sequence.", gefunden im EBI accession no. EM_EST:GW883681 Database accession no. GW883681
- BURGOS J M ET AL: "Direct molecular profiling of minicircle signatures and lineages of Trypanosoma cruzi bloodstream populations causing congenital Chagas disease", INTERNATIONAL JOURNAL OF PARASITOLOGY, PERGAMON PRESS, GB, Bd. 37, Nr. 12, 1. Oktober 2007 (2007-10-01), Seiten 1319-1327, XP026865144, ISSN: 0020-7519 [gefunden am 2007-08-24] & DATABASE EMBL [Online] 11. September 2006 (2006-09-11), "Trypanosoma cruzi clone Si.I-3 kinetoplastid minicircle, partial sequence.", gefunden im EBI accession no. EM_STD:DQ873372 Database accession no. DQ873372
- Anonymous: "GSN:BBT95049", , 12 March 2015 (2015-03-12), XP055598909, Retrieved from the Internet: URL:http://ibis/exam/dbfetch.jsp?id=GSN:BB T95049 [retrieved on 2019-06-24]
- Anonymous: "GS_NUC_ALERT:US2013267429.374977", , 10 October 2013 (2013-10-10), XP055598778, Retrieved from the Internet: URL:http://ibis/exam/dbfetch.jsp?id=GS_NUC _ALERT:US2013267429.374977 [retrieved on 2019-06-24]
- Anonymous: "EM_EST:GW883681", , 5 October 2010 (2010-10-05), XP055598845, Retrieved from the Internet: URL:http://ibis/exam/dbfetch.jsp?id=EM_EST :GW883681 [retrieved on 2019-06-24]
- Anonymous: "EM_STD:DQ873372", , 1 January 2007 (2007-01-01), XP055598875, Retrieved from the Internet: URL:http://ibis/exam/dbfetch.jsp?id=EM_STD :DQ873372 [retrieved on 2019-06-24]
- Anonymous: "GSN:ADM81392", , 8 March 2001 (2001-03-08), XP055598891, Retrieved from the Internet: URL:http://ibis/exam/dbfetch.jsp?id=GSN:AD M81392 [retrieved on 2019-06-24]

## Beschreibung

Die vorliegende Erfindung betrifft Oligonucleotide und betrifft die Verwendung der Oligonucleotide für die medizinische Diagnose. Insbesondere betrifft die Erfindung Oligonucleotide, die für eine Real-Time-Polymerase-Kettenreaktion (RT-PCR) in der Diagnose der Chagas-Krankheit Verwendung finden können.

Die Chagas-Erkrankung gehört zu den parasitären Infektionen und wird durch das Protozoon *Trypanosoma cruzi (T. cruzi)* verursacht. Übertragen wird der Erreger hauptsächlich durch verschiedene blutsaugende Raubwanzen. Auch sind kongenitale Übertragungen, Übertragung durch Bluttransfusionen und Organtransplantationen, Übertragungen durch kontaminierte Lebensmittel und Übertragungen durch Laborunfälle dokumentiert.

Mehr als 150 Haus- und Wildtiere dienen als Reservoir, aber auch asymptomatisch infizierte Menschen tragen zur Verbreitung bei. 100 Millionen Menschen leben mit dem Risiko sich zu infizieren, rund 18 Millionen sind bereits infiziert. Damit steht Chagas an 6. Stelle der "Global Burden Diseases" und stellt ein großes Problem für Süd- und Mittelamerika dar. Durch vermehrte Migration infizierter Menschen in die USA und nach Spanien und Portugal mehren sich auch dort die Fälle der Chagas-Erkrankung.

Verschiedene Raubwanzen-Arten (Familie *Reduviidae,* Unterfamilie *Triatomae*) dienen als Vektoren. Sie nehmen die Erreger beim Blutsaugen auf. Die Erreger vermehren sich im Insekt und werden dann, wenn die Raubwanze Blut von einem neuen Wirt, beispielsweise von einem Menschen, saugt, mit dem Kot der Raubwanze während des Saugaktes ausgeschieden.

Der Wanzenstich ist zumeist schmerzlos, hinterläßt jedoch eine Hautläsion und einen Juckreiz. Bekämpft die/der Gestochene den Juckreiz (oft unwillkürlich) durch Reiben, werden die Erreger mit dem Wanzenkot in die Wunde eingerieben.

Auch ist eine Infektion über die Schleimhäute der Augen (Konjunktiva), der Nase oder des Mundes möglich. Im Gewebe dringen die Erreger in die Wirtszelle ein und vermehren sich durch Zweiteilung, wobei sie sog. Pseudozysten bilden. Schließlich platzt die Pseudozyste auf und setzt die Parasiten frei, welche dann durch die Blutbahn zu neuen Wirtszellen transportiert werden und so weitere Vermehrungszyklen initiieren. Bei der nächsten Blutmahlzeit infizieren sich andere Raubwanzen, wozu selbst niedrige Parasitämien ausreichen. Raubwanzen sind nachtaktive Tiere. Sie können bis zu 2 Jahre alt werden und vermehren sich durch Eiablage. Ihr Radius beträgt mehrere Hundert Meter. Alle Stadien (Adulte und Larven) und beide Geschlechter saugen Blut. Einmal infiziert, bleiben sie lebenslang infektiös.

Beim Menschen verläuft die Chagas-Erkrankung in verschiedenen Stadien. In der akuten Phase kann es zu einer lokalen Entzündungsreaktion (Chagom) mit benachbarter Lymphknotenschwellung kommen. Ist diese am Auge, findet sich das charakteristische Romaña-Zeichen, eine Konjunktivitis mit Lidödem und präaurikulärer Lymphknotenschwellung. Das Romaña-Zeichen kommt allerdings nur selten vor (<5%). Die unspezifischen Krankheitssymptome gleichen einem grippalen Infekt. Die Symptome klingen im allgemeinen spontan wieder ab, oft innerhalb von 2 bis 4 Wochen. In wenigen Fällen kommt es in diesem Stadium zu Meningoenzephalitis oder Myokarditis mit letalen Ausgang.

Danach folgt eine Latenzphase. Hier findet sich ein positiver serologischer Test und zeitweise auch eine geringe Parasitämie; diese Phase wird auch indeterminante oder chronisch indeterminante Form genannt. Normalerweise ist diese Phase beschwerdefrei, Untersuchungen zeigten hier aber schon in einigen Fällen diskrete Störungen des Nervensystems.

30 bis 40% der Betroffenen gehen dann in ein chronisches Stadium über, das mit schwerwiegende Veränderungen am Herzen (Kardiomyopathie, plötzlicher Herztod, etc.) oder am Gastrointestinaltrakt (Megacolon, Megaösophagus, etc.) einhergeht.

Zwar könnte die Chagas-Erkrankung in der akuten Phase diagnostiziert werden, da die Parasitendichte hoch ist (Ausstrich, Konzentrationsverfahren, Buffy-Coat-Verfahren, etc.), dies geschieht jedoch meist nicht, sei es, daß der Arzt gar nicht erst konsultiert wird oder die Symptome nicht im Zusammenhang mit Chagas gesehen werden. Daher wird zumeist später serologisch diagnostiziert.

Als Therapeutika stehen derzeit zwei Wirkstoffe zur Verfügung: Benznidazol (Medikamente: Ragonil ®, Radanil ®) und Nifurtimox (Medikament: Lampit ®). Wegen des etwas günstigeren Nebenwirkungsprofils ist Benznidazol die erste Wahl. Beide Wirkstoffe sind jedoch toxisch und sollten zu Beginn stationär verabreicht werden. Sie vernichten die Parasiten im Blut und sind daher besonders in der akuten Phase erfolgreich. Die Behandlungen sind langwierig (60-90 Tage für Benznidazol, 90-120 Tage für Nifurtimox). Die Vor- und Nachteile müssen insbesondere bei chronischen Infektionen vom behandelnden Arzt gut abgewägt werden. Während der Schwangerschaft dürfen sie nicht eingesetzt werden.

Um den Kreislauf der Infektionen zu durchbrechen, müssen neben Bekämpfungs- und Präventionsmaßnahmen (Verbesserung der Lebensumstände, Fumigationen, Aufklärung, etc.) auch gute diagnostische Kits und Methoden zur Verfügung stehen, um Chagas frühzeitig zu erkennen, zu behandeln und die Weitergabe durch Reservoire (Mensch und Tier) zu verhindern. Blut- und Organspender sollten auf Chagas gescreent, kongenitale Infektionen und Reaktivierungen bei Immunsuppression aufgedeckt und behandelt werden. Dazu bedarf es neuer Diagnose-Kits und -Methoden, die eine bessere Sensitivität und Spezifität aufweisen als die bereits bekannten Mittel und Verfahren.

Weiter ist es bei allen diagnostischen Ansätzen wichtig, *T. cruzi* von anderen, verwandten und z.T. apathogen Trypanosomen (wie z.B. *T. rangeli*) zu unterscheiden: Eine einer "Positiv"-Diagnose folgende Behandlung mit den o. g. Wirkstoffen ist aufgrund der Toxizität nicht vertretbar, wenn die Positiv-Diagnose fälschlicherweise auf die Erkennung eines apathogenen Erregers zurückgeht.

Bisher fehlt ein Goldstandard zur Diagnosestellung der Chagas-Krankheit. Im chronischen Stadium werden meist serologische Verfahren angewandt. Wegen der geringen Erreger-Mengen, wie sie bei chronischer oder chronisch indeterminanter Form der Chagas-Krankheit häufig sind, wurde im Stand der Technik bereits die PCR zur Diagnose der Chagas-Krankheit neben anderen Diagnose-Methoden beschrieben.

In einer großen Multicenter-Studie (A G Schijman et al., (2011), International Study to Evaluate PCR Methods for Detection of Trypanosoma cruzi DNA in Blood Samples from Chagas Disease Patients, PLoS Negl Trop Dis 5 (1): e931) wurden 48 PCR's gegeneinander getestet. Dabei kamen die Autoren zu dem Ergebnis, dass 4 PCR's als die besten deklariert wurden. Eine davon war die konventionelle Gel-PCR(LbQ IDNA). Die drei anderen waren die Sat-DNA-PCR's LbD2-Sat-DNA-PCR, LbD3- Sat-DNA-PCR und LbF1- Sat-DNA-PCR. Die letztgenannte (LbF1- Sat-DNA-PCR) wird nachfolgend bezeichnet als "TCZ-PCR".

Der von Schijmann et al. berichtete Ansatz wurde aufgenommen von "Y Qvarnström et al., (2012), Sensitive and Specific Detection of Trypanosoma cruzi DNA in Clinical Specimens using a multi-Target Real-Time PCR Approach, PLoS Negl Trop Dis 6(7),: e1689): Sie verglichen ein bereits bekannt positives Chagas-Kollektiv mit den Ergebnissen der Mini-Satelliten-TCZ-PCR, der führenden Kinetoplasten-PCR (kDNA-PCR) und einer 18s-rRNA-PCR, die als besonders spezifisch gilt. Aus den Daten können folgende Sensivitäten bzw. Spezifitäten berechnet werden:
78 % bzw. 40 % für kDNA-PCR;
63 % bzw. 100 % für TCZ-PCR;
6 % bzw. 100 % für 18s-rRNA-PCR.

Die Autoren kamen zu dem Schluss, dass nur bei denjenigen Patienten, für die in allen drei PCR's positive Testergebnisse vorlagen, eine sichere Chagas-Diagnose gestellt werden könnte. Jede Probe, die nur in kDNA-PCR und/oder TCZ-PCR ein positives Ergebnis lieferte, bedürfe einer weiteren Abklärung. Diese bleibt vor Ort jedoch meist Theorie: Zusätzliche Diagnoseschritte werden meist aus Kostengründen und/oder infolge des Fehlens von geeignetem Equipment nicht mehr durchgeführt. Damit bleibt die Diagnose in der Praxis oft unklar, und eine Therapie wird deswegen nicht durchgeführt.

Nichtsdestoweniger ergibt sich aus den Untersuchungen, die in den vorgenannten Druckschriften berichtet wurden, dass Real-Time-PCR's (RT-PCR) ein hoher Stellenwert zugemessen wird.

Eine Sonde zur Detektion von *Trypanosoma cruzi* in biologischen Proben ist in CA2736087A1 beschrieben.

Weiter sind nach wie vor die angewendeten Verfahren zur Feststellung akuter Infektionen oder kongenitaler Erkrankungen, zur Erfolgskontrolle nach einer Therapie, bei Reaktivierung unter Immunsuppression oder zur Detektion von Erreger-Reservoirs und Maßnahmenkontrollen sowie zum Screening vor Organtransplantationen unzureichend.

Im Rahmen der Erfindung wurde ein Proben-Kollektiv untersucht, das sich durch eine größere Anzahl von Probanden (1009 vs. 119 - Qvarnström, a. a. O.), das Vorhandensein aller Altersgruppen, das Vorhandensein von Gesunden sowie verschiedener Stadien der Chagas-Krankheit (akut, chronisch, chronischindeterminant, negativ) von dem Kollektiv von Qvarnström unterscheidet.

Im Rahmen der eigenen Experimente wurde insbesondere nachgewiesen, dass die kDNA-PCR zu zahlreichen falschen positiven Ergebnissen führt. Diese beruhen überwiegend auf Kreuzreaktionen mit *Trypanosoma rangeli.* Zusammenfassend kann gesagt werden, dass die derzeit leistungsfähigsten PCR's (RT-PCR's) hilfreich sind, aber nur unterstützend bei der Diagnosestellung eingesetzt werden können.

Der Grund könnte - ohne auf diese Deutung festgelegt werden zu wollen - darin liegen, dass die bisherigen (RT-) PCR's auf Strukturen der Zelle zielen, die entweder hoch konserviert sind (18s-rRNA-PCR) und damit sehr spezifisch sind, oder die in vielen Kopien in verschiedenen Parasiten-Genomen (z. B. auch Leishmanien) vorkommen (Kinetoplasten-kDNA-PCR) und damit fälschlicherweise eine hohe Sensitivität zeigen.

Ein weiteres Problem sind die geringen Erreger-Mengen, wie sie bei chronischer oder chronisch-indeterminanter Form der Chagas-Krankheit häufig sind. Schwierigkeiten bereitet auch die große genetische Variabilität der Erreger. Auch diese Faktoren erklären die Schwierigkeiten für die Sensitivitäten und Spezifitäten.

Im Rahmen der vorliegenden Erfindung wurden Proben aus einem Hochendemie-Gebiet in Kolumbien mittels Schnelltest oder ELISA, Immunfluoreszenz sowie mit den real-time-PCR's (RT-PCR's) kDNA-PCR, TCZ-PCR und 18s rRNA-PCR untersucht, die - wie oben berichtet: Schijman et al., Qvarnström et al. - derzeit zu den leistungsfähigsten PCR's gehören.

Zur weiteren Evaluation wurden ca. 87 kDNA-PCR-Amplifikationsprodukte kloniert und sequenziert.

Ausgehend von den Sequenzierungs-Ergebnissen erfolgte über BLAST im NCBI eine Zuordnung der Isolate zu den Erregern.

Die Klone für *Trypanosoma cruzi* wurden gegeneinander und gegen die Klone von *Trypanosoma rangeli* sowie gegen in der Datenbank hinterlegte Sequenzen abgeglichen.

Dabei konnte eine Region gefunden werden, in der sich die Sequenzen aller untersuchten *Trypanosoma cruzi* deutlich von den Sequenzen von *Trypanosoma rangeli* unterscheiden.

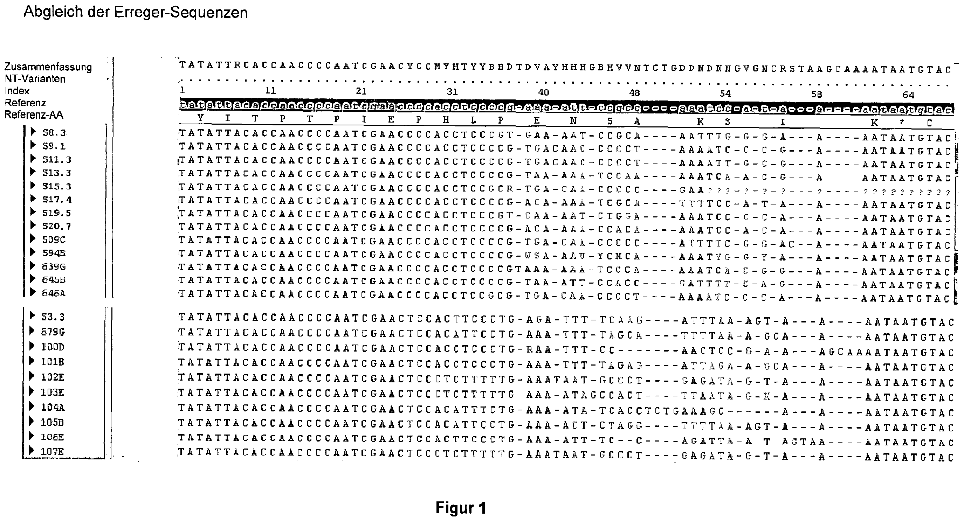

Der Abgleich der Erreger-Sequenzen ist in Figur 1 gezeigt.

Die Erfindung betrifft daher Oligonucleotide (i) mit der Sequenz SEQ ID NO 3, oder (ii) Oligonucleotide mit zu der Nucleotid-Sequenz der SEQ ID NO 3 komplementärer Nucleotid-Sequenz, oder (iü) Oligonucleotide mit hinsichtlich der Leserichtung 5'->3' zu der Nucleotid-Sequenz der SEQ ID NO 3 gegenläufiger Nucleotid-Sequenz:
cgaacccc*w ccwyc 15 SEQ ID NO3, worin
w für a oder t steht,
y für c oder t steht; und
* für ein Insert "c" steht.

Bevorzugte Ausführungsformen dieses Teils der Erfindung sind in den abhängigen Ansprüchen 2 bis 7 beansprucht und nachfolgend beispielhaft beschrieben.

Die Erfindung betrifft weiter Oligonucleotide der Sequenzen SEQ ID NO 3, SEQ ID NO 4, SEQ ID NO 5, SEQ ID NO 6, SEQ ID NO 7, SEQ ID NO 8, SEQ ID NO 9, SEC ID NO 10, SEQ ID NO 11, SEQ ID NO 13, SEQ ID NO 14, SEQ ID NO 15 und/oder SEQ ID NO 16 gemäß der nachfolgenden detaillierten Beschreibung zur Verwendung bei der Amplifikation von Krankheitserreger-Nucleotid-Sequenzen, vorzugsweise zur ausschließlichen Amplifikation von *Trypanosoma cruzi-*DNA- Sequenzen, in einer Mischung von DNA unterschiedlicher Provenienz, besonders bevorzugt durch PCR, am meisten bevorzugt durch Real-Time-PCR.

Die Erfindung betrifft weiter die Verwendung der Oligonucleotide der Sequenzen SEQ ID NO 3, SEQ ID NO 4, SEQ ID NO 5, SEQ ID NO 6, SEQ ID NO 7, SEQ ID NO 8, SEQ ID NO 9, SEC ID NO 10, SEQ ID NO 11, SEQ ID NO 13, SEQ ID NO 14, SEQ ID NO 15 und/oder SEQ ID NO 16 gemäß der nachfolgenden detaillierten Beschreibung zur Bestimmung der Präsenz oder Absenz von DNA von *Trypanosoma cruzi* im Körper eines Tieres oder Menschen, vorzugsweise im Körper eines Insekts, eines Geflügels, einer Säugers oder eines Menschen, weiter bevorzugt in einer Probe eines Körpergewebes oder einer Körperflüssigkeit eines Geflügels, eines Säugers oder eines Menschen, am meisten bevorzugt in einer Gewebeprobe oder einer Blutprobe oder einer Plasmaprobe oder einer Serumprobe eines Menschen.

Die Erfindung betrifft weiter auch die Verwendung der Oligonucleotide der Sequenzen SEQ ID NO 3, SEQ ID NO 4, SEQ ID NO 5, SEQ ID NO 6, SEQ ID NO 7, SEQ ID NO 8, SEQ ID NO 9, SEC ID NO 10, SEQ ID NO 11, SEQ ID NO 13, SEQ ID NO 14, SEQ ID NO 15 und/oder SEQ ID NO 16 gemäß der nachfolgenden detaillierten Beschreibung zur Diagnose der Chagas-Krankheit im Körper eines Tieres oder Menschen, vorzugsweise Im Körper eines Insekts, eines Geflügels, einer Säugers oder eines Menschen, weiter bevorzugt In einer Probe eines Körpergewebes oder einer Körperflüssigkeit eines Geflügels, eines Säugers oder eines Menschen, am meisten bevorzugt in einer Gewebeprobe oder einer Blutprobe oder einer Plasmaprobe oder einer Serumprobe eines Menschen.

Des Weiteren sind hier die folgenden Oligonucleotide offenbart: (i) Oligonucleotide der Sequenz SEQ ID NO 1 oder Oligonucleotide mit dazu homologen Nucleotid-Sequenzen, oder (ii) Oligonucleotide mit zu der Nucleotid-Sequenz der SEQ ID NO 1 komplementärer Nucleotid-Sequenz oder Oligonucleotide mit zu der komplementären Nucleotid-Sequenz homologen Nucleotid-Sequenzen; oder (iii) Oligonucleotide mit hinsichtlich der Leserichtung 5'->3' zu der Nucleotid-Sequenz der SEQ ID NO 1 gegenläufiger Nucleotid-Sequenz oder Oligonucleotide mit zu der gegenläufigen Nucleotid-Sequenz homologen Nucleotid-Sequenzen:
ccccwccwcc vnd 13 SEQ ID NO 1, worin
w für a oder t steht;
v für a oder c oder g steht,
d für a oder g oder t steht; und
n für a oder c oder g oder t steht.

Die Erfindung betrifft auch Oligonucleotiden der SEQ ID NO 2
ccccacctcc ccg 13 SEQ ID NO 2
und damit zusammenhängede Olignucloetide wie in Anspruch 11 beansprucht und nachfolgend beispielhaft beschrieben.

Im Rahmen der Erfindung wurden also eine für die unterscheidende Region In der DNA von *Trypanosoma cruzi* spezifische Nucleotid-Sequenz einer Sonde, die für eine Verwendung in der Real-Time-PCR geeignet ist, sowie damit kombinierbare Forward-Primer und Reverse-Primer kreiert.

Einzelheiten des durchgeführten Ansatzes ergeben sich aus den experimentellen Daten und werden dort erläutert.

Im Rahmen der vorliegenden Erfindung werden beansprucht, und werden im Rahmen bevorzugter Ausführungsformen in der Beschreibung beschrieben, Oligonucleotide, die gekennzeichnet sind durch im Einzelnen angegebene SEQ ID Nos 1 bis 16 gemäß der zur Beschreibung gehörenden Sequenzliste.

Unter "Oligonucleotiden" werden im Rahmen der Patentansprüche und der vorliegenden Beschreibung lineare Sequenzen aus bis zu 40 kovalent über Phosphorsäurediester-Brücken vom 3'-C-Atom eines Nucleotids zum 5'-C-Atom das folgenden Nucleotids miteinander gebundenen Nucleotiden verstanden. Diese werden üblicherweise (so auch in den vorliegenden Ansprüchen und in der Beschreibung) mit den kleinen Buchstaben a (für Adenin), c für Cytosin, g für Guanin und t für Thymin abgekürzt. Lineare Sequenzen werden durch die kovalente Aneinanderreihung der einzelnen Nucleotide repräsentiert. Dabei wird die angegebene Nucleotid-Sequenz immer in der Richtung 5'->3' gelesen.

Im Rahmen der vorliegenden Erfindung werden die erfindungsgemäßen Oligonucleotide immer verstanden
(i) als Oligonucleotide mit der angegebenen Sequenz SEQ ID NO, oder
(ii) als Oligonucleotide mit zu der Nucleotid-Sequenz der angegebenen SEQ ID NO komplementären Nucleotid-Sequenz; oder
(iii) als Oligonucleotide mit hinsichtlich der Leserichtung 5'->3' zu der Nucleotid-Sequenz der angegebenen SEQ ID NO gegenläufiger Nucleotid-Sequenz.

Für die Fälle von Nucleotid-Sequenzen, die zu einer angegebenen Nucleotid-Sequenz homolog sind, werden - ebenfalls wie üblich - konkrete Buchstaben für einzelne Homolog-Nucleotide verwendet, die sich im Einzelnen aus den Patentansprüchen und der nachfolgenden Beschreibung konkret ergeben. In Einzelfällen auftretende Inserts sind mit besonders definierten Zeichen (*, **, §, #) gekennzeichnet.

Die Erfindung wird nachfolgend unter Bezugnahme auf die in der Sequenzliste ebenfalls wiedergegebenen Nucleotid-Sequenzen bevorzugter Ausführungsformen beschrieben. Die Erfindung ist jedoch nicht auf die beschriebenen bevorzugten Ausführungsformen beschränkt. Diese ermöglichen in erster Linie ein besseres Verständnis der Erfindung und sollen der beispielhaften Veranschaulichung der Erfindung dienen.

Erfindungsgemäße Oligonucleotide haben in einer Ausführungsform der Erfindung die unter SEQ ID NO 3 angegebene Nucleotid-Sequenz:
cgaacccc*w ccwyc 15 SEQ ID NO 3, worin
w für a oder t steht,
y für c oder t steht; und
* für ein Insert "c" steht.

Diese Ausführungsform der Erfindung umfasst Oligonucleotide mit der angegebenen Sequenz SEQ ID NO 3 oder Oligonucleotide mit zu der Nucleotid-Sequenz der angegebenen SEQ ID NO 3 komplementären Nucleotid-Sequenz oder Oligonucleotide mit hinsichtlich der Leserichtung 5'->3' zu der Nucleotid-Sequenz der angegebenen SEQ ID NO 3 gegenläufiger Nucleotid-Sequenz.

In einer bevorzugten, da hinsichtlich der Verwendung vorteilhafte Ergebnisse liefernden Ausführungsform, die jedoch die Erfindung nicht beschränkt, betrifft die Erfindung Oligonucleotide, die umfassen die (der Sequenz mit der SEQ ID NO 3 ähnliche) Nucleotid-Sequenz der SEQ ID NO 14, oder Oligonucleotide mit zu der Nucleotid-Sequenz der SEQ ID NO 14 komplementärer Nucleotid-Sequenz, oder Oligonucleotide mit hinsichtlich der Leserichtung 5'->3' zu der Nucleotid-Sequenz der SEQ ID NO 14 gegenläufiger Nucleotid-Sequenz:
cgaaccccwc cwyc 14 SEQ ID NO 14, worin
w für a oder t steht; und
y für c oder t steht.

In einer anderen bevorzugten, da hinsichtlich der Verwendung vorteilhafte Ergebnisse liefernden Ausführungsform, die jedoch die Erfindung nicht beschränkt, betrifft die Erfindung Oligonucleotide, die umfassen die, oder sogar bestehen aus der, (der Sequenz mit der SEQ ID NO 3 und der Sequenz mit der SEQ ID NO 14 ähnlichen) Nucleotid-Sequenz der SEQ ID NO 4, oder Oligonucleotide mit zu der Nucleotid-Sequenz der SEQ ID NO 4 komplementärer Nucleotid-Sequenz, oder Oligonucleotide mit hinsichtlich der Leserichtung 5'->3' zu der Nucleotid-Sequenz der SEQ ID NO 4 gegenläufiger Nucleotid-Sequenz:
cgaacccc*a cctcc 15 SEQ ID NO 4
worin * für ein Insert "c" steht.

Noch mehr bevorzugt ist eine Ausführungsform der Erfindung mit Oligonucleotiden, die umfassen die, oder sogar bestehen aus der, (den Sequenzen mit der SEQ ID NO 3 und der SEQ ID NO 14 und der SEQ ID NO 4 ähnlichen) Nucleotid-Sequenz der SEQ ID NO 10, oder Oligonucleotide mit zu der Nucleotid-Sequenz der SEQ ID NO 10 komplementärer Nucleotid-Sequenz, oder Oligonucleotide mit hinsichtlich der Leserichtung 5'->3' zu der Nucleotid-Sequenz der SEQ ID NO 10 gegenläufiger Nucleotid-Sequenz:
cgaaccccac ctcc 14 SEQ ID NO 10

In alternativen Ausführungsformen betrifft die Erfindung Oligonucleotide, die - wie nachfolgend ersichtlich - Nucleotid-Sequenzen der vorstehenden SEQ ID Nos 3 oder 4 oder 10 oder 14 beinhalten, jedoch an ihrem 5'-Ende und/oder 3'-Ende weitere Nucleotid-Sequenzen gebunden aufweisen, oder betreffen Oligonucleotide mit zu der angegebenen Nucleotid-Sequenz komplementärer Nucleotid-Sequenz, oder Oligonucleotide mit hinsichtlich der Leserichtung 5'->3' zu der angegebenen Nucleotid-Sequenz gegenläufiger Nucleotid-Sequenz.

Diese Oligonucleotide umfassen die Nucleotid-Sequenzen der SEQ ID NO 5:
caaccccaat cgaacccc*w ccwyc vnd**d §nm#hwhy 38 SEQ ID NO 5, worin
w für a oder t steht;
m für a oder c steht;
y für c oder t steht;
v für a oder c oder g steht;
d für a oder g oder t steht;
h für a oder c oder t steht;
n für a oder c oder g oder t steht;
* für Insert c steht;
** für Insert t steht,
§ für Insert g steht; und
# für Insert c steht.

Bevorzugte Oligonucleotide der vorgenannten Gruppe umfassen Oligonucleotide der SEQ ID NO 15, oder Oligonucleotide mit zu der Nucleotid-Sequenz der SEQ ID NO 15 komplementärer Nucleotid-Sequenz, oder Oligonucleotide mit hinsichtlich der Leserichtung 5'->3' zu der Nucleotid-Sequenz der SEQ ID NO 15 gegenläufiger Nucleotid-Sequenz:
caaccccaat cgaacccc*a cctccccg**a §aa#attc 38 SEQ ID NO 15, worin
* für Insert c steht;
** für Insert t steht,
§ für Insert g steht; und
# für Insert c steht.

Ebenfalls bevorzugte Oligonucleotide der vorgenannten Gruppe umfassen Oligonucleotide der SEQ ID NO 11 oder umfassen mit zu der Nucleotid-Sequenz der SEQ ID NO: 11 komplementärer Nucleotid-Sequenz oder Oligonucleotide mit hinsichtlich der Leserichtung 5'->3' zu der Nucleotid-Sequenz der SEQ ID NO 11 gegenläufiger Nucleotid-Sequenz:
caaccccaat cgaaccccwc cwycvnddnm hwhy 34 SEQ ID NO 11, worin
w für a oder t steht;
m für a oder c steht;
y für c oder t steht;
v für a oder c oder g steht;
d für a oder g oder t steht;
h für a oder c oder t steht; und
n für a oder c oder g oder t steht.

Ebenfalls bevorzugte Oligonucleotide der vorgenannten Gruppe umfassen Oligonucleotide der SEQ ID NO 16, oder Oligonucleotide mit zu der Nucleotid-Sequenz der SEQ ID NO 16 komplementärer Nucleotid-Sequenz, oder Oligonucleotide mit hinsichtlich der Leserichtung 5'->3' zu der Nucleotid-Sequenz der SEQ ID NO 16 gegenläufiger Nucleotid-Sequenz:
caaccccaat cgaaccccac ctccccgaaa attc 34 SEQ ID NO 16.

Die letztgenannte Gruppe von Ollgonucleotiden der SEQ ID NO 16 ist aufgrund der erzielten Erkennung von *Trypanosoma cruzi*-Erregersequenzen besonders bevorzugt.

In weiteren bevorzugten Ausführungsformen der Erfindung, die allein oder mit weiteren Merkmalen der Erfindung verwirklicht werden können und die Erfindung nicht beschränken sollen, betrifft die Erfindung Oligonucleotide der vorstehend definierten SEQ ID NO 3 oder SEQ ID NO 4 oder SEQ ID NO 5 oder SEQ ID NO 10 oder SEQ ID NO 11 oder SEQ ID NO 14 oder SEQ ID NO 15 oder SEQ ID NO 16, die eine oder mehrere Markierung(en) an einem oder an beiden ihrer 5'-Enden und/oder 3'- Enden aufweisen. Solche Markierungen, die bestimmten Zwecken bei der späteren Verwendung der Oligonucleotide dienen können, sind dem Fachmann allgemein bekannt und können daher den Gegebenheiten entsprechend aus dem Stand der Technik bekannten Markierungen gewählt werden. In weiter bevorzugten Ausführungsformen ist die eine oder sind die mehreren Markierung(en) Marker und sind noch weiter bevorzugt Fluoreszenzmarker oder Quencher. Beispielhaft können an dieser Stelle bekannte Fluoreszenzmarker bzw. Quencher wie FAM oder BHQ1 verwendet werden.

Oligonucleotide der SEQ ID Nos 3, 4, 5, 10, 11, 14, 15 und/oder 16 gemäß der vorstehenden Beschreibung können beispielsweise (ohne Beschränkung) als Sonden für die PCR verwendet werden und dienen erfindungsgemäß besonders bevorzugt als Sonden für die Real-Time-PCR, ohne die Verwendung der erfindungsgemäßen Oligonucleotide auf diese Verwendung zu beschränken.

In weiteren bevorzugten Ausführungsformen der Erfindung, die allein oder mit weiteren Merkmalen der Erfindung verwirklicht werden können und die Erfindung nicht beschränken sollen, finden sich die vorstehend beschriebenen Oligonucleotide der SEQ ID NO 3 oder SEQ ID NO 4 oder SEQ ID NO 5 oder SEQ ID NO 10 oder SEQ ID NO 11 oder SEQ ID NO 14 oder SEQ ID NO 15 oder SEQ ID NO 16 gemäß der obigen detaillierten beispielhaften Beschreibung in Kombination mit einem oder mehreren weiteren Oligonucleotid(en) aus der Gruppe Forward Primer und Reverse Primer.

Weiter bevorzugt können beispielsweise Oligonucleotide der SEQ ID NO 3 oder SEQ ID NO 4 oder SEQ ID NO 5 oder SEQ ID NO 10 oder SEQ ID NO 11 oder SEQ ID NO 14 oder SEQ ID NO 15 oder SEQ ID NO 16 kombiniert werden mit einem Primer (1), beispielsweise in Form von Oligonucleotiden der Oligonucleotid-Sequenz der SEQ ID NO 6, oder von Oligonucleotiden mit zu der Nucleotid-Sequenz der SEQ ID NO 6 komplementärer Nucleotid-Sequenz , oder von Oligonucleotiden mit hinsichtlich der Leserichtung 5'->3' zu der Nucleotid-Sequenz der SEQ ID NO 6 gegenläufiger Nucleotid-Sequenz:
gcactatatt acaccaaccc c 21 SEQ ID NO 6
und/oder mit einem Primer (2), beispielsweise in Form einer Oligonucleotid-Sequenz der SEQ ID NO 7, oder von Oligonucleotiden mit zu der Nucleotid-Sequenz der SEQ ID NO 7 komplementären Nucleotid-Sequenz,; oder von Oligonucleotiden mit hinsichtlich der Leserichtung 5'->3' zu der Nucleotid-Sequenz der SEQ ID NO 7 gegenläufiger Nucleotid-Sequenz:
catgcawctc mcccgtm 17 SEQ ID NO 7; worin
w für a oder t steht; und
m für a oder c steht.

In einer bevorzugten, weil eine gute Erkennungsbasis darstellenden Ausführungsform von Kombinationen mit Oligonucleotiden der Nucleotid-Sequenz der SEQ ID NO 7, die allein oder mit anderen Merkmalen der Erfindung realisiert werden kann und die Erfindung nicht beschränken soll, oder von Oligonucleotiden mit zu der Nucleotid-Sequenz der SEQ ID NO 7 komplementären Nucleotid-Sequenz, oder von Oligonucleotiden mit hinsichtlich der Leserichtung 5'->3' zu der Nucleotid-Sequenz der SEQ ID NO 7 gegenläufiger Nucleotid-Sequenz ist die Nucleotid-Sequenz eine solche der SEQ ID NO 12:
catgcatctc ccccgta 17 SEQ ID NO 12.

Vorzugsweise ist der Primer (1) ein Forward-Primer und/oder ist der Primer (2) ein Reverse-Primer. In einer anderen Ausführungsform der Erfindung ist der Primer (2) ein Forward-Primer und/oder ist der Primer (1) ein Reverse-Primer.

Als Primer (1) und/oder Primer (2) können auch Oligonucleotide verwendet werden, die an ihrem 5'-Ende und/oder an ihrem 3'-Ende weitere Nucleotid-Sequenzen gebunden enthalten, jedoch in jedem Fall eine Oligonucleotid-Sequenz gemäß der vorstehenden SEQ ID Nos 6 und/oder 7 enthalten (oder zumindest ein Homolog davon):
Damit betrifft die Erfindung auch Kombinationen aus Oligonucleotiden der vorstehend definierten SEQ ID Nos 4, 5, 6, 7, 10, 11 und 12, die umfassen: als Primer (1) Nucleotid-Sequenzen der SEQ ID NO 8, oder Oligonucleotide mit zu der Nucleotid-Sequenz der SEQ ID NO 8 komplementärer Nucleotid-Sequenz, oder Oligonucleotide mit hinsichtlich der Leserichtung 5'->3' zu der Nucleotid-Sequenz der SEQ ID NO 8 gegenläufiger Nucleotid-Sequenz:
taaccaactg gcactatatt acaccaaccc caat 34 SEQ ID NO 8

und/oder die umfassen: als Primer (2) Nucleotid-Sequenzen der SEQ ID NO 9, oder Oligonucleotide mit zu der Nucleotid-Sequenz der SEQ ID NO 9 komplementärer Nucleotid-Sequenz, oder Oligonucleotide mit hinsichtlich der Leserichtung 5'->3' zu der Nucleotid-Sequenz der SEQ ID NO 9 gegenläufiger Nucleotid-Sequenz:
vbsbnrdwwk catgcawctc mcccgtmcat tatyt*bn 38 SEQ ID NO 9 worin
s für c oder g steht;
w für a oder t steht;
r für a oder g steht;
y für c oder t steht;
k für g oder t steht;
v für a oder c oder g steht;
d für a oder g oder t steht;
b für c oder g oder t steht;
n für a oder c oder g oder t steht; und
* für Insert "s" steht.

In einer bevorzugten, weil eine gute Erkennungsbasis darstellenden Ausführungsform von Kombinationen mit Oligonucleotiden der Nucleotid-Sequenz der SEQ ID NO 9, die allein oder mit anderen Merkmalen der Erfindung realisiert werden kann und die Erfindung nicht beschränken soll, , oder von Oligonucleotiden mit zu der Nucleotid-Sequenz der SEQ ID NO 9 komplementären Nucleotid-Sequenz, oder von Oligonucleotiden mit hinsichtlich der Leserichtung 5'->3' zu der Nucleotid-Sequenz der SEQ ID NO 9 gegenläufiger Nucleotid-Sequenz ist die Nucleotid-Sequenz eine solche der SEQ ID NO 13:
ggccagaatt catgcatctc ccccgtacat tattt*ta 38 SEQ ID NO 13,
worin * für Insert "s" steht.

Weiter bevorzugt sind auch in diesem Fall der Primer (1) ein Forward-Primer und/oder der Primer (2) ein Reverse-Primer, oder sind der Primer (2) ein Forward- Primer und/oder der Primer (1) ein Reverse-Primer.

Weiter betrifft die Erfindung gemäß einem weiteren Aspekt Oligonucleotide der Sequenzen SEQ ID NO 3, SEQ ID NO 4, SEQ ID NO 5, SEQ ID NO 6, SEQ ID NO 7, SEQ ID NO 8, SEQ ID NO 9, SEQ ID NO. 10, SEQ ID NO 11, SEQ ID NO 13, SEQ ID NO 14, SEQ ID NO 15 und SEQ ID NO 16, wie sie vorstehend im Detail beschrieben sind, zur Verwendung bei der Amplifikation von Krankheitserreger-Nucleotid-Sequenzen. Besonders haben sich die Oligonucleotide gemäß den vorstehend angegebenen SEQ ID NOs 3 bis 16 bewährt bei der ausschließlichen Amplifikation von *Trypanosoma cruzi*-DNA-Sequenzen, in einer Mischung von DNA unterschiedlicher Provenienz, besonders bevorzugt durch PCR, erfindungsgemäß am meisten bevorzugt durch Real-Time-PCR.

Die Ergebnisse der Durchführung einer Real-Time-PCR mit den erfindungsgemäßen Kombinationen aus Primern (Forward-Primern und Reverse Primern) mit einer erfindungsgemäßen Oligonucleotid-Sonde (gegebenenfalls mit Markern) gemäß der vorstehenden Beschreibung deuten - ohne auf diese Deutungen festgelegt werden zu wollen - darauf hin, dass die identifizierte Region der DNA von *Trypanosoma cruzi* ein signifikantes Unterscheidungsmerkmal zu der DNA von *Trypanosoma rangeli* ist. Kreuzreaktionen zu *Trypanosoma rangeli* werden nicht beobachtet.

Im Rahmen der Erfindung ausgetestete Stämme von *Trypanosoma cruzi* sind die Stämme *Trypanosoma cruzi CL Brenner* (Hg 39), *Trypanosoma cruzi* Typ Y (Vero) und *Trypanosoma cruzi* Brazil (HG 39). Diese wurden in den neuen Real-Time- PCR's unter Verwendung der erfindungsgemäßen Oligonucleotide detektiert. Andererseits blieben Kreuzreaktionen mit *Malaria tertiana* und *Malaria tropica* und *Leishmania brasiliensis* aus.

Die Erfindung betrifft daher auch die Verwendung der Oligonucleotide der definierten SEQ ID NOs 3 bis 11 und 13 bis 16 zur Bestimmung der Präsenz oder Absenz von DNA von *Trypanosoma cruzi* im Körper eines Tieres oder Menschen, vorzugsweise im Körper eines Insekts, eines Geflügels, einer Säugers oder eines Menschen, weiter bevorzugt in einer Probe eines Körpergewebes oder einer Körperflüssigkeit eines Geflügels, eines Säugers oder eines Menschen, am meisten bevorzugt in einer Gewebeprobe oder einer Blutprobe oder einer Plasmaprobe oder einer Serumprobe eines Menschen.

Die Erfindung betrifft weiter die Verwendung der Oligonucleotide der definierten SEQ ID NOs 3 bis 11 und 13 bis 16 zur Diagnose der Chagas-Krankheit Im Körper eines Tieres oder Menschen, vorzugsweise im Körper eines Insekts, eines Geflügels, einer Säugers oder eines Menschen, weiter bevorzugt In einer Probe eines Körpergewebes oder einer Körperflüssigkeit eines Geflügels, eines Säugers oder eines Menschen, am meisten bevorzugt in einer Gewebeprobe oder einer Blutprobe oder einer Plasmaprobe oder einer Serumprobe eines Menschen.

Wie oben beschrieben, sind hier auch die folgenden Oligonucleotide offenbart:
(i) Oligonucleotide mit der Sequenz SEQ ID NO 1 oder Oligonucleotide mit dazu homologen Nucleotid-Sequenzen, oder
(ii) Oligonucleotide mit zu der Nucleotid-Sequenz der SEQ ID NO 1 komplementärer Nucleotid-Sequenz oder Oligonucleotide mit zu der komplementären Nucleotidsequenz homologen Nucleotid-Sequenzen; oder
(iii) Oligonucleotide mit hinsichtlich der Leserichtung 5'->3' zu der Nucleotid-Sequenz der SEQ ID NO 1 gegenläufiger Nucleotid-Sequenz oder Oligonucleotide mit zu der gegenläufigen Nucleotid-Sequenz homologen Nucleotid-Sequenzen:
   ccccwccwcc vnd 13 SEQ ID NO 1, worin
   a oder t steht;
   v für a oder c oder g steht,
   d für a oder g oder t steht; und
   n für a oder c oder g oder t steht,
   welche sich zur Unterscheidung der Erreger-Sequenz von *Trypanosoma cruzi* von Sequenzen anderer *Trypanosoma* eignen, beispielsweise *Trypanosoma rangeli.*

In einer bevorzugten, weil eine gute Erkennungsbasis darstellenden Ausführungsform betrifft die Erfindung Oligonucleotide, die umfassen die, oder die bestehen aus der, Nucleotid-Sequenz SEQ ID NO 2, oder Oligonucleotide mit zu der Nucleotid-Sequenz der SEQ ID NO 2 komplementärer Nucleotid-Sequenz , oder Oligonucleotide mit hinsichtlich der Leserichtung 5'->3' zu der Nucleotid-Sequenz der SEQ ID NO 2 gegenläufiger Nucleotid-Sequenz:
ccccacctcc ccg 13 SEQ ID NO 2,
mit der eine besonders zuverlässige Unterscheidung der Erreger-Sequenz von *Trypanosoma cruzi von Trypanosoma rangeli* gelingt, im Unterschied zu den einleitend beschriebenen PCR's aus dem Stand der Technik.

### Experimenteller Teil: Teil 1 :

In dem in der nachfolgenden Tabellen-Kombination wiedergegebenen PCR-Lauf wurden verschiedene DNA-Proben verschiedener Probanden getestet. Alle Proben wurden untersucht mittels Serologie (Schnelltest [Chagas Ab Rapid von Standard Diagnostics] oder ELISA [Elisa in house des Laboratorio de Salud], Immunfluoreszenz [Immunflureszenz in house, Bernhard-Nocht Institut für Tropenmedizin]), kDNA- PCR, TCZ-PCR, 18s-rRNA-PCR.

Die in der Tabelle mit "Cruzi", "Rangeli" und "Homo s" bezeichneten Proben wurden zusätzlich sequenziert.

"Neg. Probe" 1 - 4 sind Probanden-Proben, die in allen serologischen Untersuchungen und PCR-Methoden negativ waren.

"Tulahuen" bezeichnet zwei Positiv-Kontrollen mit zwei unterschiedlichen Konzentrationen.

Für die PCR wurden folgende bevorzugten Primer verwendet;:

| | | |
|---|---|---|
| Primer 1 (Forward): (SEQ ID NO 6) | gcactatatt acaccaaccc c | (21 Nucleotide) |
| Primer 2 (Reverse): (SEQ ID NO 12) | catgcatctc ccccgta | (17 Nucleotide) |
| Sonde: (SEQ ID NO 10) | cgaaccccac ctcc | (14 Nucleotide) |

Für die PCR wurde folgender als optimal entwickelter Reaktions-Ansatz verwendet:
10 µl 2 x Master Mix (HotStarTaq Mastermix, Firma Qiagen)
1 µl Primer Forward 8 pmol (SEQ ID NO 6)
1 µl Primer Reverse 8 pmol (SEQ ID NO 12)
1 µl Sonde 2 pmol (SEQ ID NO 10)
2 µl MgCl₂ 25 mMol
3 µl Aqua dest.

Die beschriebenen 18 µl Mix wurden jeweils mit 2 µl der extrahierten DNA der zu untersuchenden Probe versetzt.

Als Positiv-Kontrolle wurde DNA von *Trypanosoma tulahuen* in gleicher Konzentration verwendet.

Das Cycler-Programm des Geräts (Rotorgene, Firma Qiagen) wurde wie folgt programmiert:
15 min 95 °C; anschließend
45 Cyclen für 15 sec 95 °C und 60 sec 60 °C; anschließend
30 sec 40 °C.

Die Ergebnisse sind den nachfolgenden Tabellen zu entnehmen.

**Tabelle 1**

| **Experiment Information** | |
|---|---|
| Run Name | Lauf definierte Proben |
| Run Start | 5/5/2015 3:31:52 AM |
| Run Finish | 5/5/2015 5:38:03 AM |
| Operator | PCR |
| Notes | |
| Run On Software Version | Rotor-Gene 2.1.0.9 |
| Run Signature | The Run Signature is valid. |
| Gain Green | 8. |
| Gain Yellow | 10. |
| Gain Orange | 10. |
| Gain Red | 10. |

**Tabelle 2**

| **Quantitation Information** | |
|---|---|
| Threshold | 0.00483 |
| Left Threshold | 1.000 |
| Standard Curve Imported | No |
| Standard Curve (1) | N/A |
| Standard Curve (2) | N/A |
| Start normalising from cycle | 1 |
| Noise Slope Correction | Yes |
| No Template Control Threshold | % 3 |
| Reaction Efficiency Threshold | Disabled |
| Normalisation Method | Dynamic Tube Normalisation |
| Digital Filter | Light |
| Sample Page | Page 1 |
| Imported Analysis Settings | |

### Ergebnisse:

**Tabelle 3**

| No. | Colour | Name | Type | Ct | Ct Comment | Given Conc (Copies) | Calc Conc (Copies) |
|---|---|---|---|---|---|---|---|
| 1 | 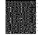 | Cruzi 1 | Unknown | 33.93 | | | |
| 2 | 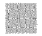 | Cruzi 1 | Unknown | 28.89 | | | |
| 3 | 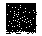 | Cruzi 1 | Unknown | 39.95 | | | |
| 4 | 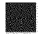 | Cruzi 2 | Unknown | 32.92 | | | |
| 5 | 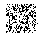 | Cruzi 2 | Unknown | 33.70 | | | |
| 6 | 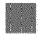 | Cruzi 2 | Unknown | 35.03 | | | |
| 7 | 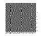 | Cruzi 3 | Unknown | 29.76 | | | |
| 8 | 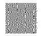 | Cruzi 3 | Unknown | 30.03 | | | |
| 9 | 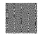 | Cruzi 3 | Unknown | 29.83 | | | |
| 10 | 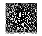 | Cruzi 4 | Unknown | 35.73 | | | |
| 11 | 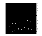 | Cruzi 4 | Unknown | 33.02 | | | |
| 12 | 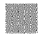 | Cruzi 4 | Unknown | 36.50 | | | |
| 13 | 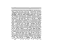 | Cruzi 5 | Unknown | 36.38 | | | |
| 14 | 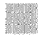 | Cruzi 5 | Unknown | 35.39 | | | |
| 15 | 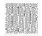 | Cruzi 5 | Unknown | 34.02 | | | |
| 16 | 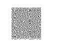 | Rangeli 1 | Unknown | | NEG (NTC) | | |
| 17 | 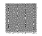 | Rangeli 1 | Unknown | | NEG (NTC) | | |
| 18 | 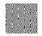 | Rangeli 1 | Unknown | | NEG (NTC) | | |
| 19 | 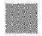 | Rangeli 2 | Unknown | | NEG (NTC) | | |
| 20 | 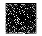 | Rangeli 2 | Unknown | | NEG (NTC) | | |
| 21 | 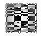 | Rangeli 2 | Unknown | | NEG (NTC) | | |
| 22 | 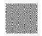 | Rangeli 3 | Unknown | | NEG (NTC) | | |
| 23 | 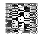 | Rangeli 3 | Unknown | | NEG (NTC) | | |
| 24 | 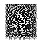 | Rangeli 3 | Unknown | | NEG (NTC) | | |
| 25 | 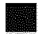 | Rangeli 4 | Unknown | | NEG (NTC) | | |
| 26 | 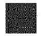 | Rangeli 4 | Unknown | | NEG (NTC) | | |
| 27 | 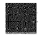 | Rangeli 4 | Unknown | | NEG (NTC) | | |
| 28 | 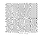 | Homo S. 1 | Unknown | | NEG (NTC) | | |
| 29 | 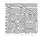 | Homo S. 1 | Unknown | | NEG (NTC) | | |
| 30 | 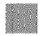 | Homo S. 1 | Unknown | | NEG (NTC) | | |
| 31 | 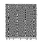 | Homo S. 2 | Unknown | | NEG (NTC) | | |
| 32 | 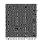 | Homo S. 2 | Unknown | | NEG (NTC) | | |
| 33 | 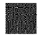 | Homo S. 2 | Unknown | 37.10 | | | |
| 34 | 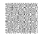 | Homo S. 3 | Unknown | | NEG (NTC) | | |
| 35 | 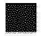 | Homo S. 3 | Unknown | | NEG (NTC) | | |
| 36 | 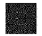 | Homo S. 3 | Unknown | | NEG (NTC) | | |
| 37 | 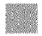 | Homo S. 4 | Unknown | | NEG (NTC) | | |
| 38 | 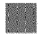 | Homo S. 4 | Unknown | | NEG (NTC) | | |
| 39 | 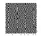 | Homo S. 4 | Unknown | | NEG (NTC) | | |
| 40 | 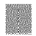 | Neg. Probe 1 | Unknown | | NEG (NTC) | | |
| 41 | 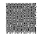 | Neg. Probe 1 | Unknown | | NEG (NTC) | | |
| 42 | 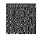 | Neg. Probe 1 | Unknown | | NEG (NTC) | | |
| 43 | 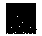 | Neg. Probe 2 | Unknown | | NEG (NTC) | | |
| 44 | 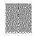 | Neg. Probe 2 | Unknown | | NEG (NTC) | | |
| 45 | 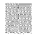 | Neg. Probe 2 | Unknown | | NEG (NTC) | | |
| 46 | 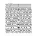 | Neg. Probe 3 | Unknown | | NEG (NTC) | | |
| 47 | 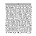 | Neg. Probe 3 | Unknown | | NEG (NTC) | | |
| 48 | 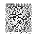 | Neg. Probe 3 | Unknown | | NEG (NTC) | | |
| 49 | 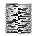 | Neg. Probe 4 | Unknown | | NEG (NTC) | | |
| 50 | 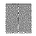 | Neg. Probe 4 | Unknown | | NEG (NTC) | | |
| 51 | 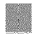 | Neg. Probe 4 | Unknown | 35.17 | | | |
| 52 | 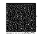 | Negativ - H2O | Unknown | | NEG (NTC) | | |
| 53 | 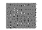 | Negativ - H20 | Unknown | | NEG (NTC) | | |
| 54 | 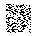 | Positiv Tulahuen 1:100 | Unknown | 23.09 | | | |
| 55 | 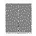 | Positiv Tuahuen 1:1000 | Unknown | 27.38 | | | |

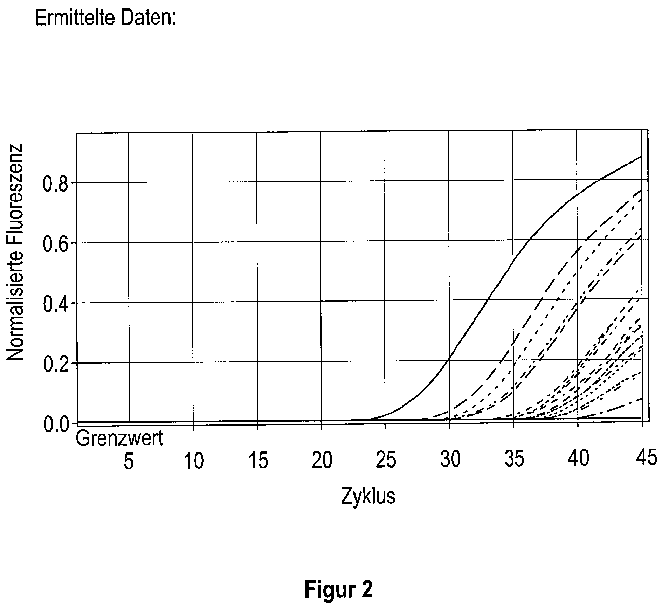

Die ermittelten Werte sind in Figur 2 in einer Graphik einander gegenübergestellt.

Wie die vorstehenden Daten, zusammen mit der Graphik von Figur 2, zeigen, wurden in allen Fällen, in denen Erregersequenzen von *Trypanosoma cruzi* bereits durch die Sequenzierung bestätigt wurden, diese in dem erwarteten CT-Bereich angezeigt und wurden damit eindeutig detektiert. Im Gegensatz dazu blieben alle (verwandten) Erregersequenzen von *Trypanosoma rangeli* im Test negativ.

### Experimenteller Teil: Teil 2:

### Sensitivität, Spezifität

Die labortechnische Diagnosestellung der Chagas-Erkrankung ist insofern eine Herausforderung, als dass es keinen Goldstandard gibt. Darüber hinaus ist die Diagnosestellung abhängig von dem Stadium der Erkrankung. So können chronische Formen bereits mit Hilfe von serologischen Verfahren (Schnelltest, Elisa, Immunfluoreszenz, etc.) gut festgestellt werden, akute, indeterminante oder kongenitale Formen sowie Rezidive oder die Kontrolle einer stattgehabten Therapie aber nicht. Im Gegensatz zu den indirekten serologischen Nachweisverfahren empfehlen sich hier die Direktnachweise des Erregers z.B. mittels RT-PCR.

Viele Ansätze sind diesbezüglich versucht und ausgewertet worden (s. Veröffentlichungen Schijman et al, Qvarnström et al und andere). Beispielhaft für viele PCRs stellten Qvarnström et al für drei der führenden PCRs Sensitivitäten und Spezifitäten dar:

| | **kDNA-PCR** | **18S rRNA PCR** | **TCZ-PCR** |
|---|---|---|---|
| **Sensitivität** | 78% | 6% | 63% |
| **Spezifität** | 40% | 100% | 100% |

(modifiziert nach Qvarnström et al, 2012)

Bemerkenswert in diesem Zusammenhang ist das untersuchte Studienkollektiv (119), das überwiegend aus Erwachsenen mit bekannter Chagas-Erkrankung bestand, welche unter Immunsuppression (nach Organtransplantation, AIDS) reaktiviert worden war, oder kongenital oder durch Laborunfälle erworben war. Bei vielen aus diesem Kollektiv waren hohe Parasitämien zu erwarten. Trotzdem kommen die Autoren zu der Schlußfolgerung, daß mit diesen PCRs zwar eine diagnostische Unterstützung stattfinden, aber keine zuverlässige Diagnose gestellt werden kann, es sei denn, die Probe zeigte sich in allen drei PCRs positiv.

Im Rahmen einer Studie in Kolumbien wurden wir auf das Problem der Chagas-Erkrankung aufmerksam. Wir haben insgesamt 1009 Proben aus einem Hochendemiegebiet auf Chagas untersucht.

Aus diesem Kollektiv, bestehend aus allen Altersklassen, Gesunden, akuten, chronischen und chronisch-indeterminanten Chagase-Erkrankten, wurden ca. 100 PCRpositive Proben sequenziert. Da alle TCZ und 18S rRNA PCR positiven Proben auch kDNA PCR positiv waren, wurde hier das Amplifikat der kDNA PCR kloniert und sequenziert. In 87 Fällen sind Klonierung und Sequenzierung gelungen. Es fanden sich 65 sequentiell gesicherte Nachweise für *Trypanosoma cruzi,* 14 für *Trypanosoma rangeli* und 8 für *Homo sapiens.*

Auch die PCR-Amplifikate von sechs Proben der neu entwickelten PCR wurden sequenziert. Alle zeigten einen positiven Nachweis für *T.cruzi.* Eine dieser positiv sequenzierten Proben war nur in der neuen PCR positiv und wurde von keiner der anderen drei PCRs detektiert.

Ausgehend von diesen Ergebnissen haben wir die Sensitivitäten und Spezifiäten für das sequenzierte Kollektiv für alle PCRs zu gleichen Bedingungen berechnet und in der nachfolgenden Tabelle aufgelistet:

| | **kDNA-PCR** | **18S rRNA PCR** | **TCZ-PCR** | ***Neu entwickelte PCR*** |
|---|---|---|---|---|
| **Sensitivität** | 89,2% | 1,5% | 20,5% | *92,3%* |
| **Spezifität** | 22,7% | 100% | 100% | *100%* |

Die guten Spezifitäten der 18S rRNA und TCZ PCR konnten - wie zuvor bei Qvarnström et al beschrieben - auch hier bestätigt werden. Allerdings zeigten sich die Sensitivitäten geringer (viele falsch negative Ergebnisse). Grund hierfür könnten die unterschiedlichen Kollektive sein. Bei Reaktivierungen z.B sind die Parasitämien meist hoch, bei einer Querschnittsbevölkerung mit vorwiegend chronischen/chronisch indeterminanten Verläufen ist die Anzahl der Parasiten jedoch eher gering.

Die kDNA-PCR zeigt eine gute Sensitivität, liegt jedoch in der Spezifität sehr niedrig. Ein Grund hierfür sind viele falsch positive Ergebnisse, die zumeist auf eine Kreuzreaktion mit *T. rangeli* zurückzuführen sind. *T. rangeli* gilt als naher Verwandter zu *T. cruzi,* wird jedoch im Gegensatz zu *T. cruzi* als apathogen angesehen. Die neu entwickelte PCR zeigt als einzige sowohl gute Sensitivitäten als auch Spezifitäten. In dieser Gruppe wurde keine Probe als falsch positiv klassifiziert und alle negativen als richtig negativ erkannt.

### Inter- und Intra-Assay-Daten

Für den Interassay wurde die Positivkontrolle Tulahuen in einer Verdünnung von 1 : 100 verwendet und an drei verschiedenen Tagen an 10 Proben pro Lauf getestet (22.-24.6.15). Der Variationskoeffizienswert belief sich auf 1,86%.

Für den Intraassay wurden 20 Proben der Positivkontrolle Tulahuen jeweils 1 : 100 und 1 : 10.000 verdünnt in einem Lauf gestestet. Der Variationskoeffizienswert belief sich auf 1,7% für die Verdünnung von 1 : 100 und auf 2,4% für die 1 : 10.000 Verdünnung.

### Primer-Dimer-Lauf

In einem Primer-Dimer-Lauf (auch Wasserlauf genannt) wurden nur Primer, Sonde, Magnesiumchlorid, Puffer und Wasser angesetzt, um ggf. Interaktionen der einzelnen Komponenten des Kits zu erkennen. Diese traten in keiner der getesteten 30 Proben auf.

### Definiertes Plasmid zur Quantifizierung

Mit Hilfe eines definierten Plasmids wurde eine Standardkurve zur Ermittlung der Quantität der Parasitämie erstellt. Dazu wurde eine bereits bekannt positive Probe amplifiziert, kloniert und sequenziert, gleichzeitig wurde die Plasmidkonzentration gemessen. Nach erneuter Bestätigung der Sequenz als *T. cruzi* und einem Meßergebnis von 10¹¹ Plasmiden / µl in der Probe konnte über eine Verdünnungsreihe im Dreifachansatz die Standarkurve ermittelt werden. Die Messung der Verdünnungsreihe sowie die Erstellung der Standardkurve erfolgte sowohl für die kDNA-PCR (modifiziert von Qvarnström et al) als auch für die neue PCR.

Im Anschluss wurden zwei bekannt positive Proben in logarithmischen Verdünnungsstufen in beiden oben genannten PCRs getestet.

Als Nachweisgrenze wurde in der Veröffentlichung von Schijman et al (2011) 5 x 10⁻³ Parasiten/ml beschrieben. In unseren Untersuchungen stellte sich für die kDNA PCR (modifiziert von Qvarnström et al) eine Nachweisgrenze von 3 x 10⁻² Parasiten/ml heraus.

Die neu entwickelte PCR hat einen Detektionsgrenze von 3 x 10⁻⁴ Erreger/ml.

Damit kann die neu entwickelte PCR selbst geringe Parasitämien nachweisen, womit die Möglichkeit besteht, die Diagnostik bei akuten, kongenitalen, chronisch-indeterminanten Infektionen sowie Therapie-Erfolgskontrollen, Reaktivierungen unter Immunsuppression, Screening vor Organtransplantationen oder Blutabgaben und Bekämpfungsmaßnahmen (z. B. Vektor-/Reservoirkontrolle usw.) zu verbessern.

## Patentansprüche

1. Oligonucleotide
(i) mit der Sequenz der SEQ ID NO 3, oder
(ii) mit zu der Nucleotid-Sequenz der SEQ ID NO 3 komplementärer Nucleotid-Sequenz, oder
(iii) mit hinsichtlich der Leserichtung 5' -> 3' zu der Nucleotid-Sequenz der SEQ ID NO 3 gegenläufiger Nucleotid-Sequenz:
cgaacccc*w ccwyc 15 SEQ ID NO 3, worin
w für a oder t steht,
y für c oder t steht; und
* für ein Insert "c" steht.

2. Oligonucleotide nach Anspruch 1,
umfassend die, oder bestehend aus der, Nucleotid-Sequenz der SEQ ID NO 14, oder Oligonucleotide mit zu der Nucleotid-Sequenz der SEQ ID NO 14 komplementärer Nucleotid-Sequenz, oder Oligonucleotide mit hinsichtlich der Leserichtung 5' -> 3' zu der Nucleotid-Sequenz der SEQ ID NO 14 gegenläufiger Nucleotid-Sequenz:
cgaaccccwc cwyc 14 SEQ ID NO 14, worin
w für a oder t steht,
y für c oder t steht; oder
umfassend die, oder bestehend aus der, Nucleotid-Sequenz der SEQ ID NO 4, oder Oligonucleotide mit zu der Nucleotid-Sequenz der SEQ ID NO 4 komplementärer Nucleotid-Sequenz, oder Oligonucleotide mit hinsichtlich der Leserichtung 5' -> 3' zu der Nucleotid-Sequenz der SEQ ID NO 4 gegenläufiger Nucleotid-Sequenz:
cgaacccc*a cctcc 15 SEQ ID NO 4,
worin * für ein Insert "c" steht; vorzugsweise
umfassend die, oder bestehend aus der, Nucleotid-Sequenz der SEQ ID NO 10, oder Oligonucleotide mit zu der Nucleotid-Sequenz der SEQ ID NO 10 komplementärer Nucleotid-Sequenz, oder Oligonucleotide mit hinsichtlich der Leserichtung 5' -> 3' zu der Nucleotid-Sequenz der SEQ ID NO 10 gegenläufiger Nucleotid-Sequenz:
cgaaccccac ctcc 14 SEQ ID NO 10.

3. Oligonucleotide nach Anspruch 1 oder 2,
umfassend die Nucleotid-Sequenz der SEQ ID NO 5, oder Oligonucleotide mit zu der Nucleotid-Sequenz der SEQ ID NO 5 komplementärer Nucleotid-Sequenz, oder Oligonucleotide mit hinsichtlich der Leserichtung 5' -> 3' zu der Nucleotid-Sequenz der SEQ ID NO 5 gegenläufiger Nucleotid-Sequenz:
caaccccaat cgaacccc*w ccwycvnd**d §nm#hwhy 38 SEQ ID NO 5 worin
w für a oder t steht;
m für a oder c steht;
y für c oder t steht;
v für a oder c oder g steht;
d für a oder g oder t steht;
h für a oder c oder t steht;
n für a oder c oder g oder t steht;
* für Insert "c" steht;
** für Insert "t" steht,
§ für Insert "g" steht; und
# für Insert "c" steht; vorzugsweise
umfassend die Nucleotid-Sequenz der SEQ ID NO 11, oder Oligonucleotide mit zu der Nucleotid-Sequenz der SEQ ID NO 11 komplementärer Nucleotid-Sequenz, oder Oligonucleotide mit hinsichtlich der Leserichtung 5' -> 3' zu der Nucleotid-Sequenz der SEQ ID NO 11 gegenläufiger Nucleotid-Sequenz:
caaccccaat cgaaccccwc cwycvnddnm hwhy 34 SEQ ID NO 11, worin
w für a oder t steht;
m für a oder c steht;
y für c oder t steht;
v für a oder c oder g steht;
d für a oder g oder t steht;
h für a oder c oder t steht;
n für a oder c oder g oder t steht; oder
umfassend die Nucleotid-Sequenz der SEQ ID NO 15, oder Oligonucleotide mit zu der Nucleotid-Sequenz der SEQ ID NO 15 komplementärer Nucleotid-Sequenz, oder Oligonucleotide mit hinsichtlich der Leserichtung 5' -> 3' zu der Nucleotid-Sequenz der SEQ ID NO 15 gegenläufiger Nucleotid-Sequenz:
caaccccaat cgaacccc*a cctccccg**a §aa#attc 38 SEQ ID NO 15, worin
* für Insert "c" steht;
** für Insert "t" steht,
§ für Insert "g" steht; und
# für Insert "c" steht; vorzugsweise
umfassend die Nucleotid-Sequenz der SEQ ID NO 16, oder Oligonucleotide mit zu der Nucleotid-Sequenz der SEQ ID NO 16 komplementärer Nucleotid-Sequenz, oder Oligonucleotide mit hinsichtlich der Leserichtung 5' -> 3' zu der Nucleotid-Sequenz der SEQ ID NO 16 gegenläufiger Nucleotid-Sequenz:
caaccccaat cgaaccccac ctccccgaaa attc 34 SEQ ID NO 16.

4. Oligonucleotide der SEQ ID NO 3 oder SEQ ID NO 4 oder SEQ ID NO 10 oder SEQ ID NO 11 nach einem oder mehreren der Ansprüche 1 bis 3, aufweisend eine oder mehrere Markierung(en) an einem oder an beiden ihrer 5'-Enden und/oder 3'-Enden, vorzugsweise worin die eine oder die mehreren Markierung(en) Marker sind, weiter bevorzugt worin die eine oder die mehreren Markierung(en) Fluoreszenzmarker oder Quencher ist/sind.

5. Oligonucleotide der SEQ ID NO 3 oder SEQ ID NO 4 oder SEQ ID NO 5 oder SEQ ID NO 10 oder SEQ ID NO 11 oder SEQ ID NO 14 oder SEQ ID NO 15 oder SEQ ID NO 16 nach einem oder mehreren der Ansprüche 1 bis 4 in Kombination mit einem oder mehreren weiteren Oligonucleotid(en) aus der Gruppe Forward Primer und Reverse Primer.

6. Oligonucleotide der SEQ ID NO 3 oder SEQ ID NO 4 oder SEQ ID NO 5 oder SEQ ID NO 10 oder SEQ ID NO 11 oder SEQ ID NO 14 oder SEQ ID NO 15 oder SEQ ID NO 16 nach Anspruch 5, worin ein Primer (1) umfasst: eine Oligonucleotid-Sequenz der SEQ ID NO 6, oder Oligonucleotide mit zu der Nucleotid-Sequenz der SEQ ID NO 6 komplementärer Nucleotid-Sequenz, oder Oligonucleotide mit hinsichtlich der Leserichtung 5' -> 3' zu der Nucleotid-Sequenz der SEQ ID NO 6 gegenläufiger Nucleotid-Sequenz:
gcactatatt acaccaaccc c 21 SEQ ID NO 6
und/oder worin ein Primer (2) umfasst: eine Oligonucleotid-Sequenz der SEQ ID NO 7, oder Oligonucleotide mit zu der Nucleotid-Sequenz der SEQ ID NO 7 komplementärer Nucleotid-Sequenz, oder Oligonucleotide mit hinsichtlich der Leserichtung 5' -> 3' zu der Nucleotid-Sequenz der SEQ ID NO 7 gegenläufiger Nucleotid-Sequenz:
catgcawctc mcccgtm 17 SEQ ID NO 7; worin
w für a oder t steht; und
m für a oder c steht; und
vorzugsweise umfasst: Oligonucleotide der Nucleotid-Sequenz der SEQ ID NO 12, oder Oligonucleotide mit zu der Nucleotid-Sequenz der SEQ ID NO 12 komplementärer Nucleotid-Sequenz, oder Oligonucleotide mit hinsichtlich der Leserichtung 5' -> 3' zu der Nucleotid-Sequenz der SEQ ID NO 12 gegenläufiger Nucleotid-Sequenz: catgcatctc ccccgta 17 SEQ ID NO 12;
weiter vorzugsweise worin der Primer (1) ein Forward-Primer und/oder worin der Primer (2) ein Reverse-Primer ist; oder worin der Primer (2) ein Forward-Primer und/oder worin der Primer (1) ein Reverse-Primer ist.

7. Oligonucleotide nach einem der Ansprüche 5 oder 6, umfassend als Primer (1) Nucleotid-Sequenzen der SEQ ID NO 8, oder Oligonucleotide mit zu der Nucleotid-Sequenz der SEQ ID NO 8 komplementärer Nucleotid-Sequenz, oder Oligonucleotide mit hinsichtlich der Leserichtung 5' -> 3' zu der Nucleotid-Sequenz der SEQ ID NO 8 gegenläufiger Nucleotid-Sequenz:
taaccaactg gcactatatt acaccaaccc caat 34 SEQ ID NO 8
und/oder umfassend als Primer (2) Nucleotid-Sequenzen der SEQ ID NO 9, oder Oligonucleotide mit zu der Nucleotid-Sequenz der SEQ ID NO 9 komplementärer Nucleotid-Sequenz, oder Oligonucleotide mit hinsichtlich der Leserichtung 5' -> 3' zu der Nucleotid-Sequenz der SEQ ID NO 9 gegenläufiger Nucleotid-Sequenz:
vbsbnrdwwk catgcawctc mcccgtmcat tatyt*bn 38 SEQ ID NO 9 worin
s für c oder g steht,
w für a oder t steht;
r für a oder g steht;
y für c oder t steht;
k für g oder t steht;
v für a oder c oder g steht;
d für a oder g oder t steht;
b für c oder g oder t steht;
n für a oder c oder g oder t steht; und
* für Insert "s" steht; und
vorzugsweise umfassend: Oligonucleotide der Nucleotid-Sequenz SEQ ID NO 13, oder Oligonucleotide mit zu der Nucleotid-Sequenz der SEQ ID NO 13 komplementären Nucleotid-Sequenz, oder Oligonucleotide mit hinsichtlich der Leserichtung 5' -> 3' zu der Nucleotid-Sequenz der SEQ ID NO 13 gegenläufiger Nucleotid-Sequenz:
ggccagaatt catgcatctc ccccgtacat tattt*ta 38 SEQ ID NO 13,
worin * für Insert "s" steht;
weiter vorzugsweise worin der Primer (1) ein Forward-Primer und/oder worin der Primer (2) ein Reverse-Primer ist; oder worin der Primer (2) ein Forward-Primer und/oder worin der Primer (1) ein Reverse-Primer ist.

8. Oligonucleotide der Sequenzen SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 13, SEQ ID NO 14, SEQ ID NO 15 und/oder SEQ ID NO 16 nach einem oder mehreren der Ansprüche 1 bis 7 zur Verwendung bei der Amplifikation von Krankheitserreger-Nucleotid-Sequenzen, vorzugsweise zur ausschließlichen Amplifikation von *Trypanosoma cruzi-*DNA-Sequenzen, in einer Mischung von DNA unterschiedlicher Provenienz, besonders bevorzugt durch PCR, am meisten bevorzugt durch Real-Time-PCR.

9. Verwendung der Oligonucleotide der Sequenzen SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 13, SEQ ID NO 14, SEQ ID NO 15 und/oder SEQ ID NO 16 nach einem oder mehreren der Ansprüche 1 bis 8 zur Bestimmung der Präsenz oder Absenz von DNA von *Trypanosoma cruzi* in einer Probe eines Körpergewebes oder einer Körperflüssigkeit eines Geflügels, eines Säugers oder eines Menschen, am meisten bevorzugt in einer Gewebeprobe oder einer Blutprobe oder einer Plasmaprobe oder einer Serumprobe eines Menschen.

10. Verwendung der Oligonucleotide der Sequenzen SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 13, SEQ ID NO 14, SEQ ID NO 15 und/oder SEQ ID NO 16 nach einem oder mehreren der Ansprüche 1 bis 8 zur Diagnose der Chagas-Krankheit in einer Probe eines Körpergewebes oder einer Körperflüssigkeit eines Geflügels, eines Säugers oder eines Menschen, am meisten bevorzugt in einer Gewebeprobe oder einer Blutprobe oder einer Plasmaprobe oder einer Serumprobe eines Menschen.

11. Oligonucleotide, bestehend aus der Nucleotid-Sequenz SEQ ID NO 2, oder Oligonucleotide mit zu der Nucleotid-Sequenz der SEQ ID NO 2 komplementärer Nucleotid-Sequenz, oder Oligonucleotide mit hinsichtlich der Leserichtung 5' -> 3' zu der Nucleotid-Sequenz der SEQ ID NO 2 gegenläufiger Nucleotid-Sequenz: ccccacctcc ccg 13 SEQ ID NO 2.

12. Verwendung eines Oligonucleotids nach Anspruch 11 zur Unterscheidung der Erreger-Sequenz von *Trypanosoma cruzi* von Sequenzen anderer *Trypanosoma,* vorzugsweise zur Unterscheidung der Erreger-Sequenz von *Trypanosoma cruzi* von der Sequenz von *Trypanosoma rangeli.*

## Claims

1. Oligonucleotides
(i) having the sequence of SEQ ID NO 3, or
(ii) having a nucleotide sequence complementary to the nucleotide sequence of SEQ ID NO 3, or
(iii) having, in reading direction 5' -> 3', a nucleotide sequence having an opposite reading direction to the nucleotide sequence of SEQ ID NO 3:
cgaacccc*w ccwyc 15 SEQ ID NO 3, wherein
w represents a or t,
y represents c or t; und
* represents an insert "c".

2. The oligonucleotides according to claim 1,
comprising, or consisting of, the nucleotide sequence of SEQ ID NO 14; or an oligonucleotide having a nucleotide sequence complementary to the nucleotide sequence of SEQ ID NO 14; or an oligonucleotide having, in reading direction 5' -> 3', a nucleotide sequence having an opposite reading direction to the nucleotide sequence of SEQ ID NO 14:
cgaaccccwc cwyc 14 SEQ ID NO 14, wherein
w represents a or t,
y represents c or t; or
comprising, or consisting of, the nucleotide sequence of SEQ ID NO 4; or an oligonucleotide having a nucleotide sequence complementary to the nucleotide sequence of SEQ ID NO 4; or an oligonucleotide having, in reading direction 5' -> 3', a nucleotide sequence having an opposite reading direction to the nucleotide sequence of SEQ ID NO 4:
cgaacccc*a cctcc 15 SEQ ID NO 4,
wherein * represents an insert "c"; preferably
comprising, or consisting of, the nucleotide sequence of SEQ ID NO 10; or an oligonucleotide having a nucleotide sequence complementary to the nucleotide sequence of SEQ ID NO 10; or an oligonucleotide having, in reading direction 5' -> 3', a nucleotide sequence having an opposite reading direction to the nucleotide sequence of SEQ ID NO 10:
cgaaccccac ctcc 14 SEQ ID NO 10.

3. The oligonucleotides according to claim 1 or 2,
comprising, or consisting of, the nucleotide sequence of SEQ ID NO 5; or an oligonucleotide having a nucleotide sequence complementary to the nucleotide sequence of SEQ ID NO 5; or an oligonucleotide having, in reading direction 5' -> 3', a nucleotide sequence having an opposite reading direction to the nucleotide sequence of SEQ ID NO 5:
caaccccaat cgaacccc*w ccwycvnd**d §nm#hwhy 38 SEQ ID NO 5 wherein
w represents a or t;
m represents a or c;
y represents c or t;
v represents a or c or g;
d represents a or g or t;
h represents a or c or t;
n represents a or c or g or t;
* represents insert "c";
** represents insert "t",
§ represents insert "g"; and
# represents insert "c"; preferably
comprising, or consisting of, the nucleotide sequence of SEQ ID NO 11; or an oligonucleotide having a nucleotide sequence complementary to the nucleotide sequence of SEQ ID NO 11; or an oligonucleotide having, in reading direction 5' -> 3', a nucleotide sequence having an opposite reading direction to the nucleotide sequence of SEQ ID NO 11:
caaccccaat cgaaccccwc cwycvnddnm hwhy 34 SEQ ID NO 11, wherein
w represents a or t;
m represents a or c;
y represents c or t;
v represents a or c or g;
d represents a or g or t;
h represents a or c or t;
n represents a or c or g or t; or
comprising, or consisting of, the nucleotide sequence of SEQ ID NO 15; or an oligonucleotide having a nucleotide sequence complementary to the nucleotide sequence of SEQ ID NO 15; or an oligonucleotide having, in reading direction 5' -> 3', a nucleotide sequence having an opposite reading direction to the nucleotide sequence of SEQ ID NO 15:
caaccccaat cgaacccc*a cctccccg**a §aa#attc 38 SEQ ID NO 15, wherein
* represents insert "c";
** represents insert "t",
§ represents insert "g"; and
# represents insert "c"; preferably
comprising, or consisting of, the nucleotide sequence of SEQ ID NO 16; or an oligonucleotide having a nucleotide sequence complementary to the nucleotide sequence of SEQ ID NO 16; or an oligonucleotide having, in reading direction 5' -> 3', a nucleotide sequence having an opposite reading direction to the nucleotide sequence of SEQ ID NO 16:
caaccccaat cgaaccccac ctccccgaaa attc 34 SEQ ID NO 16.

4. Oligonucleotides of SEQ ID NO 3 or SEQ ID NO 4 or SEQ ID NO 10 or SEQ ID NO 11 according to one or more of claims 1 to 3, having one or more marking(s) at one or both of their 5' ends and/or 3' ends, preferably wherein the one or more marking(s) are markers, further preferably wherein the one or more marking(s) is/are fluorescence markers or quenchers.

5. Oligonucleotides of SEQ ID NO 3 or SEQ ID NO 4 or SEQ ID NO 5 or SEQ ID NO 10 or SEQ ID NO 11 or SEQ ID NO 14 or SEQ ID NO 15 or SEQ ID NO 16 according to one or more of claims 1 to 4 in combination with one or more further oligonucleotide(s) selected from forward primers and reverse primers.

6. Oligonucleotides of SEQ ID NO 3 or SEQ ID NO 4 or SEQ ID NO 5 or SEQ ID NO 10 or SEQ ID NO 11 or SEQ ID NO 14 or SEQ ID NO 15 or SEQ ID NO 16 according to claim 5, wherein a primer (1) comprises: an oligonucleotide sequence of SEQ ID NO 6, or oligonucleotides having a nucleotide sequence complementary to the nucleotide sequence of SEQ ID NO 6; or oligonucleotides having, in reading direction 5' -> 3', a nucleotide sequence having an opposite reading direction to the nucleotide sequence of SEQ ID NO 6:
gcactatatt acaccaaccc c 21 SEQ ID NO 6
and/or wherein a primer (2) comprises: an oligonucleotide sequence of SEQ ID NO 7, or oligonucleotides having a nucleotide sequence complementary to the nucleotide sequence of SEQ ID NO 7; or oligonucleotides having, in reading direction 5' -> 3', a nucleotide sequence having an opposite reading direction to the nucleotide sequence of SEQ ID NO 7:
catgcawctc mcccgtm 17 SEQ ID NO 7; wherein
w represents a or t; and
m represents a or c; and
preferably comprises: oligonucleotides having the nucleotide sequence of SEQ ID NO 12, or oligonucleotides having a nucleotide sequence complementary to the nucleotide sequence of SEQ ID NO 12; or oligonucleotides having, in reading direction 5' -> 3', a nucleotide sequence having an opposite reading direction to the nucleotide sequence of SEQ ID NO 12:
catgcatctc ccccgta 17 SEQ ID NO 12;
further preferably wherein primer (1) is a forward primer and/or wherein primer (2) is a reverse primer; or wherein primer (2) is a forward primer and/or wherein primer (1) is a reverse primer.

7. Oligonucleotides according to one of claims 5 or 6, comprising as the primer (1) nucleotide sequences of SEQ ID NO 8, or oligonucleotides having a nucleotide sequence complementary to the nucleotide sequence of SEQ ID NO 8, or oligonucleotides having, in reading direction 5' -> 3', a nucleotide sequence having an opposite reading direction to the nucleotide sequence of SEQ ID NO 8:
taaccaactg gcactatatt acaccaaccc caat 34 SEQ ID NO 8
and/or comprising as the primer (2) nucleotide sequences of SEQ ID NO 9, or oligonucleotides having a nucleotide sequence complementary to the nucleotide sequence of SEQ ID NO 9; or oligonucleotides having, in reading direction 5' -> 3', a nucleotide sequence having an opposite reading direction to the nucleotide sequence of SEQ ID NO 9:
vbsbnrdwwk catgcawctc mcccgtmcat tatyt*bn 38 SEQ ID NO 9 wherein
s represents c or g,
w represents a or t;
r represents a or g;
y represents c or t;
k represents g or t;
v represents a or c or g;
d represents a or g or t;
b represents c or g or t;
n represents a or c or g or t; and
* represents Insert "s"; and
preferably comprising: oligonucleotides having the nucleotide sequence of SEQ ID NO 13, or oligonucleotides having a nucleotide sequence complementary to the nucleotide sequence of SEQ ID NO 13; or oligonucleotides having, in reading direction 5' -> 3', a nucleotide sequence having an opposite reading direction to the nucleotide sequence of SEQ ID NO 13:
ggccagaatt catgcatctc ccccgtacat tattt*ta 38 SEQ ID NO 13,
wherein * represents insert "s";
further preferably wherein primer (1) is a forward primer and/or wherein primer (2) is a reverse primer; or wherein primer (2) is a forward primer and/or wherein primer (1) is a reverse primer.

8. Oligonucleotides of the sequences of SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 13, SEQ ID NO 14, SEQ ID NO 15 and/or SEQ ID NO 16 according to one or more of claims 1 to 7 for use in amplifying pathogen nucleotide sequences, preferably for the exclusive amplification of *Trypanosoma cruzi* DNA sequences in a mixture of DNA of various origin, particularly preferred by PCR, most preferably by real time PCR.

9. Use of the oligonucleotides of the sequences of SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 13, SEQ ID NO 14, SEQ ID NO 15 and/or SEQ ID NO 16 according to one or more of claims 1 to 8 for determining the presence of absence of DNA of *Trypanosoma cruzi* in a sample of a body tissue or a body fluid of a poultry, a mammal or a human most preferably in a tissue sample or a blood sample or a plasma sample or a serum sample of a human.

10. Use of the oligonucleotides of the sequences of SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 13, SEQ ID NO 14, SEQ ID NO 15 and/or SEQ ID NO 16 according to one or more of claims 1 to 8 for diagnosing Chagas' disease in a sample of a body tissue or a body fluid of a poultry, a mammal or a human most preferably in a tissue sample or a blood sample or a plasma sample or a serum sample of a human.

11. Oligonucleotides consisting of the nucleotide sequence of SEQ ID NO 2, or oligonucleotides having a nucleotide sequence complementary to the nucleotide sequence of SEQ ID NO 2; or oligonucleotides having, in reading direction 5' -> 3', a nucleotide sequence having an opposite reading direction to the nucleotide sequence of SEQ ID NO 2:
ccccacctcc ccg 13 SEQ ID NO 2.

12. Use of an oligonucleotide according to claim 11 for distinguishing the pathogen sequence of *Trypanosoma cruzi* from sequences of other *Trypanosoma,* preferably for distinguishing the pathogen sequence of *Trypanosoma cruzi* from the sequence of *Trypanosoma range*/*i.*

## Revendications

1. Oligonucléotides
(i) ayant la séquence SEQ ID NO 3, ou
(ii) ayant une séquence nucléotidique complémentaire de la séquence nucléotidique SEQ ID NO 3 ; ou
(iii) ayant une séquence nucléotidique inverse de la séquence nucléotidique SEQ 10 ID NO 3 par rapport au sens de lecture 5' -> 3' :
cgaacccc*w ccwyc 15 SEQ ID NO 3, dans laquelle
w représente a ou t,
y représente c ou t ; et
* représente un insert « c ».

2. Oligonucléotides selon la revendication 1,
comprenant ou étant composés de la séquence nucléotidique SEQ ID NO 14, ou des oligonucléotides ayant une séquence nucléotidique complémentaire de la séquence nucléotidique SEQ ID NO 14, ou des oligonucléotides ayant une séquence nucléotidique inverse de la séquence nucléotidique SEQ ID NO 14 par rapport au sens de lecture 5' -> 3' :
cgaaccccwc cwyc 14 SEQ ID NO 14, dans laquelle
w représente a ou t,
y représente c ou t ; ou
comprenant ou étant composés de la séquence nucléotidique SEQ ID NO 4, ou des oligonucléotides ayant une séquence nucléotidique complémentaire de la séquence nucléotidique SEQ ID NO 4, ou des oligonucléotides ayant une séquence nucléotidique inverse de la séquence nucléotidique SEQ ID NO 4 par rapport au sens de lecture 5' -> 3' :
cgaacccc*a cctcc 15 SEQ ID NO 4,
dans laquelle * représente un insert « c » ; de préférence
comprenant ou étant composés de la séquence nucléotidique SEQ ID NO 10, ou des oligonucléotides ayant une séquence nucléotidique complémentaire de la séquence nucléotidique SEQ ID NO 10, ou des oligonucléotides ayant une séquence nucléotidique inverse de la séquence nucléotidique SEQ ID NO 10 par rapport au sens de lecture 5' -> 3' :
cgaaccccac ctcc 14 SEQ ID NO 10.

3. Oligonucléotides selon la revendication 1 ou 2,
comprenant la séquence nucléotidique SEQ ID NO 5, ou des oligonucléotides ayant une séquence nucléotidique complémentaire de la séquence nucléotidique SEQ ID NO 5, ou des oligonucléotides ayant une séquence nucléotidique inverse de la séquence nucléotidique SEQ ID NO 5 par rapport au sens de lecture 5' -> 3' :
caaccccaat cgaacccc*w ccwycvnd**d §nm#hwhy 38 SEQ ID NO 5 dans laquelle
w représente a ou t ;
m représente a ou c ;
y représente c ou t ;
v représente a ou c ou g ;
d représente a ou g ou t ;
h représente a ou c ou t ;
n représente a ou c ou g ou t ;
* représente un insert « c » ;
** représente un insert « t »,
§ représente un insert « g » ; et
# représente un insert « c » ; de préférence
comprenant la séquence nucléotidique SEQ ID NO 11, ou des oligonucléotides ayant une séquence nucléotidique complémentaire de la séquence nucléotidique SEQ ID NO 11, ou des oligonucléotides ayant une séquence nucléotidique inverse de la séquence nucléotidique SEQ ID NO 11 par rapport au sens de lecture 5' -> 3' :
caaccccaat cgaaccccwc cwycvnddnm hwhy 34 SEQ ID NO 11, dans laquelle
w représente a ou t ;
m représente a ou c ;
y représente c ou t ;
v représente a ou c ou g ;
d représente a ou g ou t ;
h représente a ou c ou t ;
n représente a ou c ou g ou t ; ou
comprenant la séquence nucléotidique SEQ ID NO 15, ou des oligonucléotides ayant une séquence nucléotidique complémentaire de la séquence nucléotidique SEQ ID NO 15, ou des oligonucléotides ayant une séquence nucléotidique inverse de la séquence nucléotidique SEQ ID NO 15 par rapport au sens de lecture 5' -> 3' :
caaccccaat cgaacccc*a cctccccg**a §aa#attc 38 SEQ ID NO 15, dans laquelle
* représente un insert « c » ;
** représente un insert « t »,
§ représente un insert « g » ; et
# représente un insert « c » ; de préférence
comprenant la séquence nucléotidique SEQ ID NO 16, ou des oligonucléotides ayant une séquence nucléotidique complémentaire de la séquence nucléotidique SEQ ID NO 16, ou des oligonucléotides ayant une séquence nucléotidique inverse de la séquence nucléotidique SEQ ID NO 16 par rapport au sens de lecture 5' -> 3' :
caaccccaat cgaaccccac ctccccgaaa attc 34 SEQ ID NO 16.

4. Oligonucléotides de la SEQ ID NO 3 ou de la SEQ ID NO 4 ou de la SEQ ID NO 10 ou de la SEQ ID NO 11 selon l'une ou plusieurs des revendications 1 à 3, comprenant un ou plusieurs marquage(s) à l'une ou aux deux de leurs extrémités 5' et/ou extrémités 3', de préférence dans lesquels lesdits un ou lesdits plusieurs marquage(s) sont des marqueurs, plus préférentiellement dans lesquels lesdits un ou lesdits plusieurs marquage(s) sont des marqueurs fluorescents ou des extincteurs.

5. Oligonucléotides de la SEQ ID NO 3 ou de la SEQ ID NO 4 ou de la SEQ ID NO 5 ou de la SEQ ID NO 10 ou de la SEQ ID NO 11 ou de la SEQ ID NO 14 ou de la SEQ ID NO 15 ou de la SEQ ID NO 16 selon l'une ou plusieurs des revendications 1 à 4 en combinaison avec un ou plusieurs autre(s) oligonucléotide(s) du groupe des amorces sens et des amorces anti-sens.

6. Oligonucléotides de la SEQ ID NO 3 ou de la SEQ ID NO 4 ou de la SEQ ID NO 5 ou de la SEQ ID NO 10 ou de la SEQ ID NO 11 ou de la SEQ ID NO 14 ou de la SEQ ID NO 15 ou de la SEQ ID NO 16 selon la revendication 5, dans lesquels une amorce comprend (1) : une séquence oligonucléotidique SEQ ID NO 6, ou des oligonucléotides ayant une séquence nucléotidique complémentaire de la séquence nucléotidique SEQ ID NO 6, ou des oligonucléotides ayant une séquence nucléotidique inverse de la séquence nucléotidique SEQ ID NO 6 par rapport au sens de lecture 5' -> 3' :
gcactatatt acaccaaccc c 21 SEQ ID NO 6
et/ou dans lesquels une amorce (2) comprend : une séquence oligonucléotidique SEQ ID NO 7, ou des oligonucléotides ayant une séquence nucléotidique complémentaire de la séquence nucléotidique SEQ ID NO 7, ou des oligonucléotides ayant une séquence nucléotidique inverse de la séquence nucléotidique SEQ ID NO 7 par rapport au sens de lecture 5' -> 3' :
catgcawctc mcccgtm 17 SEQ ID NO 7 ; dans laquelle
w représente a ou t ; et
m représente a ou c ; et
comprend de préférence : des oligonucléotides de la séquence nucléotidique SEQ ID NO 12, ou des oligonucléotides ayant une séquence nucléotidique complémentaire de la séquence nucléotidique SEQ ID NO 12, ou des oligonucléotides ayant une séquence nucléotidique inverse de la séquence nucléotidique SEQ ID NO 12 par rapport au sens de lecture 5' -> 3' : catgcatctc ccccgta 17 SEQ ID NO 12 ;
plus préférentiellement dans lesquels l'amorce (1) est une amorce sens et/ou dans lesquels l'amorce (2) est une amorce anti-sens ; ou dans lesquels l'amorce (2) est une amorce sens et/ou dans lesquels l'amorce (1) est une amorce anti-sens.

7. Oligonucléotides selon l'une des revendications 5 ou 6, comprenant comme amorce (1) des séquences nucléotidiques SEQ ID NO 8, ou des oligonucléotides ayant une séquence nucléotidique complémentaire de la séquence nucléotidique SEQ ID NO 8, ou des oligonucléotides ayant une séquence nucléotidique inverse de la séquence nucléotidique SEQ ID NO 8 par rapport au sens de lecture 5' -> 3' :
taaccaactg gcactatatt acaccaaccc caat 34 SEQ ID NO 8
et/ou comprenant comme amorce (2) des séquences nucléotidiques SEQ ID NO 9, ou des oligonucléotides ayant une séquence nucléotidique complémentaire de la séquence nucléotidique SEQ ID NO 9, ou des oligonucléotides ayant une séquence nucléotidique inverse de la séquence nucléotidique SEQ ID NO 9 par rapport au sens de lecture 5' -> 3' :
vbsbnrdwwk catgcawctc mcccgtmcat tatyt*bn 38 SEQ ID NO 9 dans laquelle
s représente c ou g,
w représente a ou t ;
r représente a ou g ;
y représente c ou t ;
k représente g ou t ;
v représente a ou c ou g ;
d représente a ou g ou t ;
b représente c ou g ou t ;
n représente a ou c ou g ou t ; et
* représente un insert « s » ; et
comprenant de préférence : des oligonucléotides de la séquence nucléotidique SEQ ID NO 13, ou des oligonucléotides ayant une séquence nucléotidique complémentaire de la séquence nucléotidique SEQ ID NO 13, ou des oligonucléotides ayant une séquence nucléotidique inverse de la séquence nucléotidique SEQ ID NO 13 par rapport au sens de lecture 5' -> 3' : ggccagaatt catgcatctc ccccgtacat tattt*ta 38 SEQ ID NO 13,
dans laquelle * représente un insert « s » ;
plus préférentiellement dans lesquels l'amorce (1) est une amorce sens et/ou dans lesquels l'amorce (2) est une amorce anti-sens ; ou dans lesquels l'amorce (2) est une amorce sens et/ou dans lesquels l'amorce (1) est une amorce anti-sens.

8. Oligonucléotides des séquences SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 13, SEQ ID NO 14, SEQ ID NO 15 et/ou SEQ ID NO 16 selon l'une ou plusieurs des revendications 1 à 7 destinés à être utilisés pour l'amplification de séquences nucléotidiques pathogènes, de préférence pour l'amplification exclusive de séquences d'ADN de *Trypanosoma cruzi,* dans un mélange d'ADN de provenance différente, de façon plus préférée par PCR, le plus préférentiellement par PCR en temps réel.

9. Utilisation des oligonucléotides des séquences SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 13, SEQ ID NO 14, SEQ ID NO 15 et/ou SEQ ID NO 16 selon l'une ou plusieurs des revendications 1 à 8 pour déterminer la présence ou l'absence d'ADN de *Trypanosoma cruzi* dans un échantillon d'un tissu corporel ou d'un fluide corporel d'une volaille, d'un mammifère ou d'un être humain, le plus préférentiellement dans un échantillon de tissu, un échantillon de sang, un échantillon de plasma ou un échantillon de sérum d'un être humain.

10. Utilisation des oligonucléotides des séquences SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 13, SEQ ID NO 14, SEQ ID NO 15 et/ou SEQ ID NO 16 selon l'une ou plusieurs des revendications 1 à 8 pour diagnostiquer la maladie de Chagas dans un échantillon d'un tissu corporel ou d'un fluide corporel d'une volaille, d'un mammifère ou d'un être humain, le plus préférentiellement dans un échantillon de tissu, un échantillon de sang, un échantillon de plasma ou un échantillon de sérum d'un être humain.

11. Oligonucléotides constitués de la séquence nucléotidique SEQ ID NO 2, ou d'oligonucléotides ayant une séquence nucléotidique complémentaire de la séquence nucléotidique SEQ ID NO 2, ou d'oligonucléotides ayant une séquence nucléotidique inverse de la séquence nucléotidique SEQ ID NO 2 par rapport au sens de lecture 5' -> 3' :
ccccacctcc ccg 13 SEQ ID NO 2.

12. Utilisation d'un oligonucléotide selon la revendication 11 pour distinguer la séquence pathogène de *Trypanosoma cruzi* des séquences d'autres *Trypanosoma,* de préférence pour distinguer la séquence pathogène de *Trypanosoma cruzi* de la séquence de *Trypanosoma rangeli.*


### SEQUENCE LISTING

<110> Kann Simone
<120> Oligonucleotide und deren Verwendung
<130> P 3000 WO
<150> DE102015113038.6
   <151> 2015-08-07
<150> DE102015111267.1
   <151> 2015-07-13
<160> 16
<170> BiSSAP 1.3.6
<210> 1
   <211> 13
   <212> DNA
   <213> künstliche Sequenz
<220>
   <223> Oligonucleotid
<400> 1
   ccccwccwcc vnd 13
<210> 2
   <211> 13
   <212> DNA
   <213> künstliche Sequenz
<220>
   <223> Oligonucleotid
<400> 2
   ccccacctcc ccg 13
<210> 3
   <211> 15
   <212> DNA
   <213> künstliche Sequenz
<220>
   <223> Oligonucleotid Sonde
<400> 3
   cgaacccccw ccwyc 15
<210> 4
   <211> 15
   <212> DNA
   <213> künstliche Sequenz
<220>
   <223> Oligonucleotid Sonde
<400> 4
   cgaaccccca cctcc 15
<210> 5
   <211> 38
   <212> DNA
   <213> künstliche Sequenz
<220>
   <223> Oligonucleotid Sonde
<400> 5
   caaccccaat cgaacccccw ccwycvndtd gnmchwhy 38
<210> 6
   <211> 21
   <212> DNA
   <213> künstliche Sequenz
<220>
   <223> Oligonucleotid Primer
<400> 6
   gcactatatt acaccaaccc c 21
<210> 7
   <211> 17
   <212> DNA
   <213> künstliche Sequenz
<220>
   <223> Oligonucleotid Primer
<400> 7
   catgcawctc mcccgtm 17
<210> 8
   <211> 34
   <212> DNA
   <213> künstliche Sequenz
<220>
   <223> Oligonucleotid Primer
<400> 8
   taaccaactg gcactatatt acaccaaccc caat 34
<210> 9
   <211> 38
   <212> DNA
   <213> künstliche Sequenz
<220>
   <223> Oligonucleotid Primer
<400> 9
   vbsbnrdwwk catgcawctc mcccgtmcat tatytsbn 38
<210> 10
   <211> 14
   <212> DNA
   <213> künstliche Sequenz
<220>
   <223> Oligonucleotid Sonde
<400> 10
   cgaaccccac ctcc 14
<210> 11
   <211> 34
   <212> DNA
   <213> künstliche Sequenz
<220>
   <223> Oligonucleotid Sonde
<400> 11
   caaccccaat cgaaccccwc cwycvnddnm hwhy 34
<210> 12
   <211> 17
   <212> DNA
   <213> künstliche Sequenz
<220>
   <223> Oligonucleotid Primer
<400> 12
   catgcatctc ccccgta 17
<210> 13
   <211> 38
   <212> DNA
   <213> künstliche Sequenz
<220>
   <223> Oligonucleotid Primer
<400> 13
   ggccagaatt catgcatctc ccccgtacat tatttsta 38
<210> 14
   <211> 14
   <212> DNA
   <213> künstliche Sequenz
<220>
   <223> Oligonucleotid Sonde
<400> 14
   cgaaccccwc cwyc 14
<210> 15
   <211> 38
   <212> DNA
   <213> künstliche Sequenz
<220>
   <223> Oligonucleotid Sonde
<400> 15
   caaccccaat cgaaccccca cctccccgta gaacattc 38
<210> 16
   <211> 34
   <212> DNA
   <213> künstliche Sequenz
<220>
   <223> Oligonucleotid Sonde
<400> 16
   caaccccaat cgaaccccac ctccccgaaa attc 34